(19)

**Europäisches Patentamt**
**European Patent Office**
**Office européen des brevets**

(11) **EP 4 400 549 A1**

(12) **EUROPEAN PATENT APPLICATION**

(43) Date of publication:
**17.07.2024 Bulletin 2024/29**

(21) Application number: **24151099.9**

(22) Date of filing: **10.01.2024**

(51) International Patent Classification (IPC):
***C09C 1/42*** *(2006.01)*      ***A61K 33/06*** *(2006.01)*
***A61K 33/12*** *(2006.01)*

(52) Cooperative Patent Classification (CPC):
**C09C 1/42; A61K 33/06; A61K 33/12**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **11.01.2023 IT 202300000237**

(71) Applicants:
• **Consiglio Nazionale Delle Ricerche**
**00185 Roma (IT)**
• **Laviosa Chimica Mineraria S.p.A.**
**57123 Livorno (IT)**

(72) Inventors:
• **MENICAGLI, Elena**
**57016 Rosignano Solvay (IT)**
• **CAVALLINI, Irene**
**57124 Livorno (IT)**
• **AVANTAGGIATO, Giuseppina**
**70010 Capurso (IT)**
• **D ASCANIO, Vito**
**70124 Bari (IT)**
• **GRECO, Donato**
**70126 Bari (IT)**

(74) Representative: **Cattaneo, Elisabetta et al**
**Notarbartolo & Gervasi S.p.A.**
**Viale Achille Papa, 30**
**20149 Milano (IT)**

(54) **ORGANOPHILIC SMECTITE FUNCTIONALIZED WITH CHOLINE FOR THE ADSORPTION OF MYCOTOXINS**

(57) The invention relates to a process for the preparation of a new organophilic smectite comprising the following steps: a) supplying a sodium smectite of sedimentary geological origin; b) treating said smectite with $H_2SO_4$ in an amount in the range from 10% to 30% by weight with respect to the smectite dry weight, at a temperature in the range from 20°C to 50°C, to obtain an activated smectite; c) treating said activated smectite with at least one modifying agent consisting of a quaternary ammonium salt of formula $[(CH_3)_3N^+(CH_2)_2OH]$ $X^-$, where $X^-$ is a monovalent anion, where said modifying agent is at a concentration in the range from 30 to 130 meq/100g to obtain a functionalized smectite; and d) extruding the functionalized smectite to reach an extrudate temperature equal to or higher than 45°C, and drying it at a temperature in the range from 30°C to 80°C. The smectite of the invention acts as an additive and/or supplement capable of sequestering, *in vitro,* a broad spectrum of mycotoxins and reducing, *in vivo,* their gastro-intestinal absorption and toxicity (mycotoxicosis).

**EP 4 400 549 A1**

**Description**

**FIELD OF THE INVENTION**

The invention concerns a process for the preparation of an organophilic smectite functionalized with choline and the smectite obtainable from the same process. The smectite of the invention is capable of sequestering a wide spectrum of structurally different mycotoxins and may therefore be used as an additive and/or supplement to food and feed to reduce exposure to mycotoxins and prevent their toxicity (mycotoxicosis).

**STATE OF THE ART**

Mycotoxins are fungal metabolites that are toxic to vertebrates and other groups of animals even at low concentrations. Aflatoxins are a group of mycotoxins produced by fungi of the *Aspergillus* genus and have been found to be both toxic and carcinogenic to humans and animals. Aflatoxin B1 (AFB1) and mixtures of B1, B2, G1, G2 and M1 aflatoxins are carcinogenic to humans and were included in Group 1 by the IARC (International Agency for Research on Cancer).

The diseases (**mycotoxicoses**) caused by these toxins are quite varied and involve a wide range of susceptible animal species, including humans. Due to toxicity and diffusion in agri-food products, aflatoxin B1 (AFB1), deoxynivalenol (DON), zearalenone (ZEA), ochratoxin A (OTA), fumonisin B1 (FB1) and T-2 and HT-2 toxins are of particular interest. The extent of damage each toxin can cause is highly species-dependent. These mycotoxins are often found simultaneously in foodstuffs (co-contamination) due to environmental and substrate conditions. Due to co-contamination problems, it is very likely that humans and animals are exposed to mixtures rather than single compounds, which can produce toxicological interactions with synergistic effects. One of the strategies to reduce exposure to mycotoxins is to reduce their bioavailability by including inert, indigestible materials with adsorbent activity in the feed.

The presence of these additives has the effect of reducing the gastrointestinal absorption of mycotoxins and their distribution to the blood and target organs. The use and placing on the European market of these feed additives is regulated by Commission Regulation (EC) No. 386/2009, which introduced a new functional group in the category of technological feed additives called "*substances for reduction of the contamination of feed by mycotoxins: substances that can suppress or reduce the absorption, promote the excretion of mycotoxins or modify their mode of action*" (CE, 386/2009). According to EFSA guidelines published in July 2010, the effectiveness of these additives has to be demonstrated both through *in vitro* and *in vivo* studies. *In vitro* evaluations are considered a key step in the development and quality control of feed additives and are useful as a screening method for potential detoxifying agents.

A variety of substances have been studied as potential mycotoxin adsorbent agents, including aluminosilicates, activated carbons, indigestible complex carbohydrates (cellulose, polysaccharides found in the cell walls of yeast and bacteria such as glucomannans, peptidoglycans, and others), and synthetic polymers such as cholestyramine and polyvinylpyrrolidone and derivatives. Silicate minerals are the largest class of mycotoxin sequestering agents, and most studies on the mitigation of mycotoxicoses using adsorbent agents have focused on aluminosilicates. In this group, there are 2 important subclasses: the phyllosilicate subclass and the tectosilicate subclass. Phyllosilicates include bentonites (smectites, montmorillonite), kaolinites, illite. Tectosilicates include zeolites.

Bentonite is a clay mineral from the smectite family, mainly made up of montmorillonite. The crystallographic base of montmorillonite is typical of phyllosilicates, consisting of layers of AIX octahedra (X = oxygen or hydroxy) interposed between two layers of $SiO_4$ tetrahedra.

Smectites are a group of minerals that show expandability, due to absorption of both water and organic molecules within the structural layers, and also notable cation exchange properties. The clay mineral making them up in the crystalline state derives from devitrification and consequent chemical alteration of glass of magmatic origin, generally tuff or volcanic ash. The nature and volcanic origins of bentonite deposits give rise to often highly heterogeneous varieties of the mineral.

Sodium and calcium bentonites, and acidic bentonites are thus formed.

Bentonites have been shown to act as enterosorbents that very strongly and selectively bind aflatoxins in the gastrointestinal (GI) tract of humans and animals decreasing their bioavailability and associated toxicity. However, these clays have the limitation of being quite effective against aflatoxins while not being able to prevent the toxic effects of *Fusarium* mycotoxins (such as fumonisins, trichothecenes or ZEA) and ochratoxins. Likewise, the efficacy of other mineral adsorbents against fumonisins, OTA and ZEA can range from high to limited, and the efficacy against trichothecenes (DON, T-2, HT-2) is close to zero. Recent studies have shown that the adsorption of mycotoxins other than aflatoxins can be improved by coating some minerals, including clays, with long-chain quaternary ammonium salts, such as quaternary alkyl-arylamines. Clays, in their natural state, have a hydrophilic surface. Modification with cationic surfactants improves the surface hydrophobic character and leads to surface charge reversal from negative to positive. Organophilic clay can therefore be obtained by simple cation exchange, which serves to fix the surfactant molecules in the interlamellar space. These alterations in the clay surface properties result in an increase in hydrophobicity and interlamellar space; montmorillonites and clinoptilolites thus modified have proven to be particularly effective, *in vitro,* for ZEA and OTA. However, these compounds have not found any practical applications. *In vivo* studies with laboratory animals have, in fact, shown the potential toxicity of some of these types of clay and the surfactants used to produce them are not allowed in the food sector, including animal feed.

In addition to the aforementioned organoclays, only activated carbons have been selected as broad-spectrum adsorbent agents; however, their efficacy in reducing mycotoxin toxicity in animals has not been confirmed and their use in animal feed remains somewhat questionable due to their ability to sequester essential nutritional principles.

Recently, to overcome the toxicity problem of organoclays intercalated with long-chain quaternary ammonium salts, new materials have been developed by treating clays with amphipathic biomolecules such as lecithin. *In vitro* studies demonstrated the ability of these bio-organoclays to adsorb AFB1 and ZEA, as well as a series of synthetic contaminants. Although no *in vivo* efficacy data have been reported, the ability of bio-organoclays to reduce the bioavailability of these contaminants in humans and animals has been speculated, and their use in the food sector in emergency situations, such as natural disasters or epidemics, has been suggested. A widespread and deeply felt need in the feed, livestock and food sectors is therefore the development of new materials, to be used as additives, in particular for feed, capable of adsorbing/sequestering a wide spectrum of biological contaminants present in food and feed, such as the aforementioned mycotoxins.

Bentonites as adsorbent agents are widely used as feed components (technological additives), in the food (supplements) and pharmaceutical (both as certified medical devices and excipients) sectors. Clay feed additives were initially used to prevent cracking and improve the technological properties of animal feed. Clay-based products are also present on the European market and certified as natural bioregulators to protect human health by preventing and alleviating symptoms caused by exposure to pollutants. These products act like a sponge, selectively absorbing pollutants in the gastro-intestinal tract, and then expelling them naturally from the body. Clays and minerals, in fact, are not digestible and are expelled naturally, together with the pollutants they have absorbed, through the feces through the intestine, without burdening the metabolism.

The decreased toxicity of aflatoxins observed for contaminated feed treated with clay has stimulated research on clay additives since the early 1980s in order to abate all known mycotoxins.

The publication "Simultaneous Detoxification of Aflatoxin B1, Zearalenone and Deoxynivalenol by Modified Montmorillonites" by Jiaqi Mao 1,†,Ying Zhou 2,†,Guanglie Lv 1 andRenxian Zhou 1, Molecules 2022, 27(1), 315; https://doi.org/10.3390/molecules27010315 describes the treatment of a calcium smectite of the montmorillonite type (Ca-based montmorillonite, MMT) with $H_2SO_4$ and modification with organic modifiers. The acid treatment was carried out at about 90°C for 3 hours and the smectite surfaces were subsequently modified with organic compounds that more easily bound contaminants than untreated clays. Montmorillonites treated with organic cations, such as hexadecyltrimethylammonium (HDTMA) and phenyltrimethylammonium (PTMA), more effectively removed organic contaminants, such as benzene and toluene, from water compared to untreated clays. Similarly, PTMA-treated montmorillonite more effectively retained AFB1 from an aqueous cornmeal extract compared to untreated montmorillonite. However, the organic cations HDTMA and PTMA are toxic and therefore cannot be considered suitable as components of clay additives intended for animal feeding.

In "Aflatoxin Toxicity Reduction in Feed by Enhanced Binding to Surface-Modified Clay Additives", William F. Jaynes and Richard E. Zartman Toxins (Basel). 2011 June; 3(6): 551-565, doi: 10.3390/toxins3060551, the adsorption efficacy of a series of organo-clays prepared from a calcium-type montmorillonite (Novasil) is examined, albeit for aflatoxin only. In the publication the starting material is a calcium bentonite first transformed into sodium bentonite, and then treated with various modifiers, in solution, for an hour. The final products are claimed as being able to adsorb AFB1 with greater efficiency than the starting mineral.

In the patent application Pub. No: US 2021/0137971 A1 entitled "Edible enterosorbents used to mitigate acute exposures to ingestible environmental toxins following outbreaks, natural disasters and emergencies", Phillips et al. 2021, a new enterosorbent for the adsorption of mycotoxins and synthetic contaminants, obtained by treating a montmorillonite-based mineral with a strong acid and/or lecithin, is described. In the aforementioned patent application, it is specified that the acid treatment, in addition to producing a proton exchange with silicate metals, has the effect of dissolving the octahedral/tetrahedral structures typical of clay crystalline structure, to produce a high porosity compound, with an amorphous structure comparable to carbon-based materials rather than smectite. The acidic mineral is also treated with lecithin. In the patent application the capacity (especially of acid smectite) to adsorb AFB1 and ZEA, as well as a series of synthetic chemical contaminants, is demonstrated only with *in vitro* tests.

The known modified clays have therefore shown a selective capacity to adsorb mycotoxins and for some of them a certain toxicity has been reported in the literature.

The need is therefore felt to provide materials that are able to effectively adsorb/sequester not only aflatoxins, but also a broader spectrum of mycotoxins.

The object of the present invention is therefore to provide a food additive that is capable of sequestering structurally different mycotoxins and that can be included in food and feed to reduce exposure to mycotoxins and prevent the onset of mycotoxicosis.

**SUMMARY OF THE INVENTION**

In an attempt to find a product that met the needs indicated above, the inventors surprisingly found a raw material and a series of process steps that made it possible to have a new organophilic smectite capable of sequestering structurally different mycotoxins and, therefore, being able to be used as a food and feed additive.

In a first aspect, therefore, the invention concerns a process for the preparation of an organophilic smectite functionalized with choline comprising the following steps:

a) supplying a sodium smectite of sedimentary geological origin;
b) treating said smectite with $H_2SO_4$ in an amount in the range from 10% to 30% by weight with respect to the smectite dry weight and at a temperature in the range from 20°C to 50°C to obtain an activated smectite;
c) treating said activated smectite with at least one modifying agent consisting of a quaternary ammonium salt of formula $[(CH_3)_3N^+(CH_2)_2OH]$ X-, where X- is a monovalent anion,
where said modifying agent is at a concentration in the range from 30 to 130 meq/100g to obtain a functionalized smectite; and
d) extruding the functionalized smectite to reach an extrudate temperature equal to or higher than 45°C, and drying it at a temperature in the range from 30°C to 80°C.

The inventors, in fact, carried out preliminary analyses and realized that smectites of the sodium type and of sedimentary geological origin are better performing than the calcium or hydrothermal smectites of the prior art and have therefore surprisingly modified the activation and functionalization process, thus obtaining choline-functionalized organophilic smectites highly performing in the adsorption of a wide range of mycotoxins, as will be apparent from the experimental part. In particular, the inventors found that the use of low amounts of sulfuric acid, with respect to the amount of smectite, for activation before the functionalization step, allowed the structure of the mineral to be preserved, improving its adsorption performance. In this aspect, the product of the invention differed from those of the prior art.

Surprisingly, and as will be apparent from the experimental part, step b) involving treatment of said smectite with $H_2SO_4$ in an amount in the range from 10% to 30% by weight with respect to the smectite dry weight, and at a temperature in the range from 20°C at 50°C, allowed to obtain an activated smectite with an unchanged crystalline structure compared to the sodium smectite of step a).

The functionalization step with the quaternary ammonium salt of formula $[(CH_3)_3N^+(CH_2)_2OH]$ X-, where X- is a monovalent anion, allowed choline to be intercalated inside the smectite structure, which was functionalized and extruded in step d).

In another aspect, the invention therefore concerns an organophilic smectite functionalized/intercalated with choline obtainable by the process of the invention, wherein said organophilic smectite comprises at least the organic modifier consisting of choline in the mineral interlayer.

The additive developed by the inventors can therefore be classified as a "bio-organoclay" or "bio-organic clay" and has demonstrated effectiveness as a multi-mycotoxin binder, which was confirmed in animals, both laboratory animals and in livestock farming, using the biomarker approach.

Advantageously, therefore, the choline-functionalized organophilic smectites of the invention did not show selective adsorption of a single group of mycotoxins, generally aflatoxins, but showed an excellent adsorption capacity of a wide range of mycotoxins. Prior art smectites have shown selective aflatoxin adsorption capacity and little or almost no adsorption efficiency against other low-polarity and hydrophobic mycotoxins, such as zearalenones, ochratoxins, and fumonisins, while the smectites invention were found to be active also against these mycotoxins.

The organophilic smectite of the invention, with an acidic character and comprising an organic modifier consisting of choline in the mineral interlayer, was found to be active in sequestering a wide range of mycotoxins including aflatoxin B1 (AFB1), zearalenone (ZEA), ochratoxin A (OTA) and fumonisin B1 (FB1).

Under optimal conditions, the smectite of the invention as a bio-organoclay showed to be capable of simultaneously sequestering, *in vitro,* from 10 to 100%, preferably over 95%, of AFB1, FB1, OTA and ZEA over a wide range of pH values (3-9), preferably at a concentration of 1 ppm for each toxin in the pool. Preferably, the organophilic smectite of the invention is able to simultaneously sequestering AFB1, ZEA, OTA and FB1, at bio-organoclay dosages equal to or greater than 0.1% w/v, with maximum adsorption capacity values (expressed as $\mu$g of toxin adsorbed per mg of smectite) of between 30-150 $\mu$g/mg for AFB1, 20-150 $\mu$g/mg for FB1, 20-60 $\mu$g/mg for ZEA and 4-20 $\mu$g/mg for OTA.

In a further aspect, therefore, the invention concerns the use of the organophilic smectite of the invention for mycotoxin decontamination from food products, in particular feed, to prevent their toxic effects in living organisms, including laboratory animals and in livestock farming.

Living organisms in the present invention mean monogastric animals (including chickens, pigs, fish and rodents), polygastric animals (such as dairy cows, sheep, goats, etc.) and human beings.

Mycotoxins preferably comprise AFB1, ZEA, OTA and FB1.

In a further aspect, the invention relates to the use of the organophilic smectite of the invention for reducing gastro-intestinal absorption and exposure of humans and animals to mycotoxins, as measured by specific biomarkers (of exposure and/or effect) of mycotoxins.

The invention therefore concerns the choline-functionalized organophilic smectite of the invention for use in the treatment and prevention of mycotoxicosis of humans and animals.

Treatment and prevention can therefore be assessed using specific biomarkers. The markers are mainly, but not exclusively, focused on the measurement of plasma and/or urinary profiles of mycotoxins and their metabolites, and on the values of AUC (area under the curve), Cmax (maximum absorption concentration), as well as on the concentration values of mycotoxins and their metabolites in biological samples, such as biological fluids, tissues, organs, hair, feces, *etc.*.

The organophilic smectite of the invention, therefore, administered orally by addition to food or feed, at dosages equal to or greater than 0.25% w/w with respect to the weight of the food product, was able to reduce the values of mycotoxin biomarkers by 10 to 95%.

At these dosages, the use of the organophilic smectite of the invention advantageously did not show toxic side effects and did not interfere with the absorption of micronutrients, including water-soluble vitamins, fat-soluble vitamins and minerals.

At the dosages indicated above, the organophilic smectite of the invention was able to prevent mycotoxin toxicity, such as acute and chronic mycotoxicosis and the onset of cancer.

Such organophilic smectites can therefore be advantageously used as additives in foods or supplements and as animal feed additives.

In another aspect, the invention concerns a food product, a feed or a supplement comprising the smectite of the invention as an additive.

The invention will now be described in detail for illustrative purposes also with reference to the attached figures.

## DESCRIPTION OF THE FIGURES

Figure 1 shows the effect of the concentration of the choline-functionalized smectite object of the invention, on the simultaneous adsorption of aflatoxin B1 (AFB1), zearalenone (ZEA), ochratoxin A (OTA) and fumonisin B1 (FB1).

Figure 2 shows the effect of the mycotoxin concentration on the adsorption of aflatoxin B1 (AFB1), zearalenone (ZEA), ochratoxin A (OTA) and fumonisin B1 (FB1) by the organophilic smectite object of the invention (adsorption isotherms at the equilibrium).

Figure 3 shows the XRD diffraction spectra of a raw smectite (3A) and a smectite activated with 15% w/w sulfuric acid (3B).

Figure 4 shows the XRD diffraction spectra of a raw smectite (4A) and a smectite activated with sulfuric acid and functionalized with choline chloride (4B). The two diffractograms in Fig.4B relate to two replicates of the material prepared at different times.

Figure 5 shows the effect of the bio-organoclay of the invention in reducing the urinary excretion of aflatoxin M1 (AFM1) in rats treated with 30 $\mu$g of AFB1 and 125 mg of clay. Control animals received the toxin in the absence of the clay. Results are reported as mean$\pm$SD (n=6).

Figure 6 shows the effect of the bio-organoclay of the invention in reducing the urinary excretion of ZEA (and phase I metabolites) in rats treated with 300 $\mu$g of ZEA and 125 mg of clay. Control animals received the toxin in the absence of the clay. Results are reported as mean$\pm$SD (n=9).

Figure 7 shows the effect of the bio-organoclay of the invention in reducing the urinary excretion of OTA in rats treated with 125 $\mu$g of OTA and 125 mg of clay. Control animals received the toxin in the absence of the clay. Results are reported as mean$\pm$SD (n=6).

Figure 8 shows the effect of the bio-organoclay in reducing the urinary excretion of FB1 in rats treated with 3 mg of FB1 and 125 mg of clay. Control animals received the toxin in the absence of the clay. Results are shown as mean$\pm$SD (n=6).

Figure 9 shows the plasma concentration/time profile for AFB1 after administration of the toxin by intragastric bolus, alone (C1 and C2) or in association with bio-organoclay (T1 and T2), to 7 or 8 pigs per subgroup (C1: n=7; T1, C2, T2: n=8). In subgroups C1 and T1 the feed was suspended from 12 hours before to 4 hours after administration. Values represent the mean+SD.

Figure 10 shows the plasma concentration/time profile for OTA after administration of the toxin by intragastric bolus, alone (C1) or in association with bio-organoclay (T1), to 7 or 8 pigs per subgroup (C1: n= 7; T1: n=8). In subgroups C1 and T1 the feed was suspended from 12 hours before to 4 hours after administration. Values represent the mean+SD.

Figure 11 shows the plasma concentration/time profile for ZEN-GlcA after administration of ZEA by intragastric bolus, alone (C1) or in association with bio-organoclay (T1), to 7 or 8 pigs per subgroup (C1: n=7; T1: n=8). In subgroups C1 and T1 the feed was suspended from 12 hours before to 4 hours after administration. Values represent the mean+SD.

Figure 12 shows the plasma concentration/time profile for FB1 after administration of the toxin by intragastric bolus, alone (C1) or in association with bio-organoclay (T1), to 7 or 8 pigs per subgroup (C1: n= 7; T1: n=8). In subgroups C1 and T1 the feed was suspended from 12 hours before to 4 hours after administration. Values represent the mean+SD.

Figure 13 shows the serum concentration/time profiles for vitamin A (Vit A), vitamin B12 (Vit B12) and calcium (Ca), respectively, after bolus administration, to 6 pigs, of the mycotoxin mixture (AFB1, OTA, ZEN, FB1) in combination with the bio-organoclay object of the invention. Values represent the mean±SD.

## DETAILED DESCRIPTION OF THE INVENTION

The invention therefore concerns a process for the preparation of an organophilic smectite functionalized with choline comprising the following steps:

a) supplying a sodium smectite of sedimentary geological origin;
b) treating said smectite with $H_2SO_4$ in an amount in the range from 10% to 30% by weight with respect to the smectite dry weight at a temperature in the range from 20°C to 50°C to obtain an activated smectite;
c) treating said activated smectite with at least one modifying agent consisting of a quaternary ammonium salt of formula $[(CH_3)_3N^+(CH_2)_2OH]$ $X^-$, where $X^-$ is a monovalent anion,
where said modifying agent is at a concentration in the range from 30 to 130 meq/100g to obtain a functionalized smectite; and
d) extruding the functionalized smectite to reach an extrudate temperature equal to or higher than 45°C, and drying it at a temperature in the range from 30°C to 80°C.

The process of the invention involves the step a) of supplying a sodium smectite of sedimentary geological origin. The smectite is preferably in a crude form and contains from about 15% to 50% moisture, more preferably about 20%. The sodium smectite of the invention preferably contains an amount equal to or greater than 70% of montmorillonite.

Without being tied to any theory, the inventors believe that the activation yield of step b) depends on the geological origin of the smectites of the invention which should be of the sedimentary, and not hydrothermal, type.

Step b) of the invention consists in treating the sodium smectite of sedimentary geological origin with $H_2SO_4$ in an amount in the range from 10% to 30% by weight with respect to the smectite dry weight, at a temperature in the range from 20°C to 50°C, to obtain an activated smectite.

As detailed in Example 2 in the experimental part and from the mycotoxin adsorption data reported in Table 1, it can be seen that sodium smectites of sedimentary origin, if activated with sulfuric acid, produce activated smectites with higher and broader adsorbent power, compared to calcium and hydrothermal type smectites.

The activation step occurs with sulfuric acid. As described in Example 2 in the experimental part, different acids were tested at different concentrations, but sulfuric acid, in a concentration in the range from 10% to 30% by weight with respect to the smectite dry weight, was found to be the most effective in the subsequent mycotoxin adsorption step. Preferably, an amount of about 15% was found to be the most effective in improving mycotoxin adsorption.

As an example, Table 2 shows the results for mycotoxin adsorption recorded by activating a sodium smectite of sedimentary geological origin with an organic (citric) acid and an inorganic (sulfuric) acid at a concentration of 10% by weight with respect to the dry weight of smectite. As described in Example 2 in the experimental part, and from the results in Table 2, at the same acid concentration, activation with sulfuric acid was more effective in increasing the adsorbent power of native smectite. The results in Table 2 were obtained using the multi-mycotoxin adsorption test described in the experimental part (Example 2).

For the activation with sulfuric acid, an acid concentration of 15% is preferred as it significantly increases the adsorption of all toxins and, in particular, OTA, compared to lower acid concentrations. As can be seen from the results reported in Table 3 of Example 2, ZEA, OTA and FB1 adsorptions increased significantly as the concentration of sulfuric acid increased, being maximum at the acid concentration of 15%. The results in Table 3 were also obtained using the multi-mycotoxin adsorption test described in the experimental part (Example 2).

The treatment step with sulfuric acid preferably takes place for a reaction time of 3 to 7 minutes, more preferably about 5 minutes.

The treatment step with $H_2SO_4$ takes place at a temperature in the range from 20°C to 50°C, preferably around 25°C.

By using the optimal conditions treatment with sulfuric acid, *i.e.* a concentration of about 15% by weight with respect to the smectite dry weight, for a time of about 5 minutes and at a temperature of about 25°C, the activated smectite preferably adsorbed >90% of AFB1, FB1 and OTA, and >64% of ZEA regardless of the pH value of the medium.

In order to determine the relevance of the treatment temperature in the activation process, a comparison was also made with a treatment step with identical conditions except for the temperature, which was as in the prior art, *i.e.* about 60-80°C. The results are reported in Table 4 in the experimental part and were obtained using the multi-mycotoxin adsorption test described in Example 2.

As can be seen from Table 4 reported in Example 2, step b) when carried out at 25°C allowed the mycotoxins to be effectively adsorbed regardless of the pH values. Advantageously, step b) of treating the smectite with $H_2SO_4$ in an amount in the range from 10% to 30% by weight with respect to the smectite dry weight, and at a temperature in the range from 20°C to 50°C, allows to obtain an activated smectite with unaltered crystalline structure compared to raw smectite.

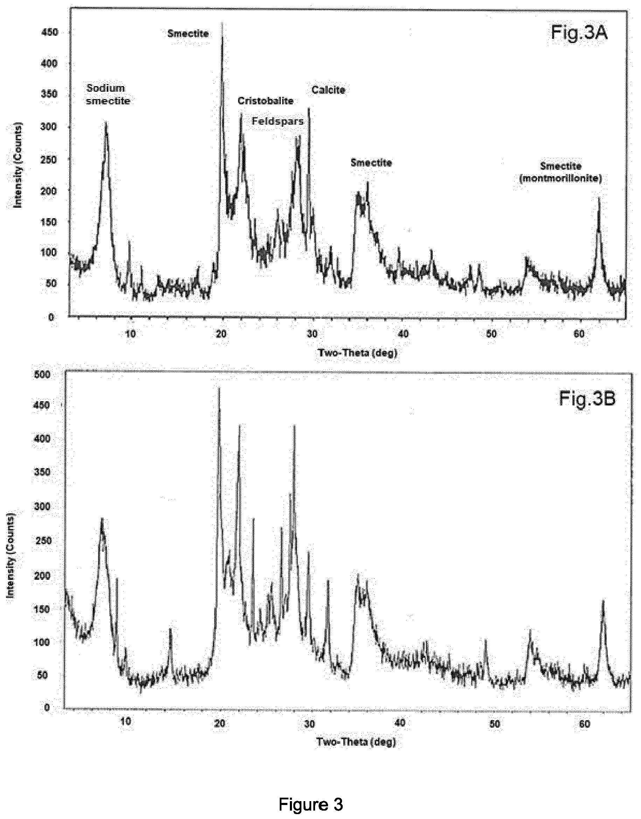

The acid treatment of step b) of the process of the invention is carried out with an acid concentration and for a time such as not to alter the crystalline structure of the starting mineral, as evidenced by spectroscopic analysis (XRD) reported in the experimental part. In the present invention, smectites acid activation is therefore carried out in "mild" conditions, with an acid concentration, at a temperature and for a time such as not to alter the crystalline structure of the starting mineral. The results of the spectroscopic (Figure 3) and mineralogical (Table 5) analyses have in fact demonstrated that the smectite of step a) and the activated smectite of step b) had a similar structure and comparable chemical-physical properties.

Step c) of the process of the invention consists in treating the activated smectite of step b) with at least one modifying agent consisting of a quaternary ammonium salt of formula $[(CH_3)_3N^+(CH_2)_2OH]$ X-, where X- is a monovalent anion, where said modifying agent is at a concentration in the range of 30 to 130 meq/100g, to obtain a functionalized smectite.

Said monovalent anion is preferably selected from the group consisting of chloride, hydroxide anion, bitartrate and dihydrogen citrate.

Step c) of the invention, in fact, made it possible to significantly improve the adsorption of ZEA and OTA, showing high efficacy also against AFB1 and FB1.

The results in Table 6 of Example 2 in the experimental part highlight that choline chloride is the most effective modifying agent for increasing the mycotoxin adsorption capacity of the final products. For the organic modifier choice, a sodium smectite of sedimentary origin (not activated) was functionalized with the modifiers listed in Table 6 including lecithin of the prior art. At the same concentration of modifying agents and experimental conditions, choline chloride was found to be significantly more effective in increasing the adsorption of ZEA, OTA and FB1 (especially at acidic pH). The results in Table 6 were obtained using the multi-mycotoxin adsorption test described in the experimental part (Example 2).

In step c) a concentration of choline chloride in the range from 30 to 130 meq/100g, preferably from 60 to 75 meq/100g, is therefore used.

In order to determine the most suitable modifier concentration range for the purpose of the invention, a native (non-activated) sedimentary sodium smectite was treated with increasing concentrations of choline chloride from 30 meq/100g to 130 meq/100g. The bio-clays were then tested for their effectiveness in binding mycotoxins at different pH values using the multi-mycotoxin adsorption assay described in Example 2. As can be seen from the results reported in Table 7 of Example 2 (experimental part), the effect of the organic modifier concentration on mycotoxin adsorption was found to be dose-dependent. The adsorption of ZEA and OTA recorded at pH 3 increased significantly as the amount of organic modifier increased, from 31 to 130 meq/100g, with an optimum at values of 65-75 meq/100g. A further increase in concentration to values above 130 meq/100g had a negative effect on toxin adsorption.

According to the invention, the modifying agent choline chloride could be used in dried or liquid form. The dried form was advantageous from an industrial point of view.

Without being tied to any theory, the inventors believe that the combination of step b) and step c), if both carried out in the sequence shown, allow for a surprising improvement in mycotoxins adsorption compared to the products of the prior art.

A comparison was therefore made between the adsorption capacities of native smectites, those of step b) and those of step c), whether subjected to the previous step b) or not.

In particular, sodium smectites of sedimentary geological origin were used and treated under the optimal conditions of step b). Step c) was also carried out according to the optimal conditions indicated above, *i.e.* choline chloride was used at a concentration in the range from 65 to 75 meq/100g.

The mycotoxin adsorption values were recorded at neutral and acidic pH using the multi-mycotoxin adsorption and analysis (UHPLC-PDA/FLD/MS) techniques described in the experimental part of Example 2. The results are reported in Table 8 of the same example.

As shown by the data in Table 8, the chemical activation of step b) of the invention followed by functionalization with choline chloride allowed to obtain a product capable of effectively and multifunctionally adsorbing different toxins, not only at gastric pH (pH=3) but also at intestinal pH (pH=7).

According to the invention, the preparation process of organophilic smectite involves a step d) of extruding the functionalized smectite to reach a temperature of the extrudate equal to or higher than 45°C and drying it in the range from 30°C to 80°C. Step d) therefore involves an extrusion process during which the extrudate should reach temperatures equal to or higher than 45°C, and subsequent drying in an oven at a temperature of about 65°C. Without being tied to any theory, the inventors believe that the extrusion step allows mechanical energy to be imparted to promote cationic exchange in the product, in order to obtain the clay modified with choline defined in the invention as organophilic smectite.

In another aspect, the invention concerns an organophilic smectite obtainable from the process of the invention, wherein said organophilic smectite comprises the organic modifier consisting of choline in the mineral interlayer. Said organophilic smectite preferably has an acidic character.

The final product of the process, i.e. the organophilic smectite, acidic and modified with the organic modifier consisting of choline in the mineral interlayer, is also called MMDA "*multi-mycotoxin detoxifying agent*".

In a further aspect, the invention concerns the use of the organophilic smectite of the invention for mycotoxin adsorption.

The inventors also tested the final product to study the mycotoxin adsorption mechanism and to determine the best adsorption conditions. In particular, the effect of adsorbent dosage, toxin concentration, pH of the medium and temperature on adsorption efficiency was studied. The main adsorption (maximum capacity, affinity, chemoadsorption) and thermodynamics parameters were determined.

As described in the experimental part (Example 3), the effect of the adsorbent concentration on the adsorption of mycotoxins was investigated through the use of equilibrium isotherms. The adsorption experiments were performed in triplicate, at gastric and intestinal pH, testing a fixed amount of toxins (1 $\mu$g/mL) with different dosages of the smectite of the invention (0.0005-0.5% w/v). The adsorption of AFB1, FB1, ZEA, and OTA was significantly affected by the dosage, and the percentage of mycotoxins removed from the buffer solutions at both pH values increased as the amount of smectite increased (Example 3).

The experimental AFB1, ZEA, OTA and FB1 adsorption values were in the range of 10 - 100% for AFB1, 3 - 100% for ZEA, 3 - 100% for OTA and 7 - 100% for FB1.

As will be apparent from the experimental part (Example 4), the effect of the pH of the medium on the adsorption of mycotoxins was investigated by testing a fixed amount of both toxins (1 $\mu$g/mL) and smectite of the invention (0.5% w/v), in buffered solutions at different pH values (3-9). Under these experimental conditions, the smectite of the invention sequestered AFB1, ZEA, OTA and FB1 to an equal extent and in all pH ranges, recording adsorption values of $\geq$95% in all cases.

The release of mycotoxins from the smectite of the invention due to a change in the pH of the medium and extraction with methanol was also determined; and the chemisorption index was calculated (Example 4).

In agreement with previous results, mycotoxin adsorption at pH=3 was 100% for all target toxins. A change in the pH of the medium (from acidic to neutral) did not release the adsorbed toxins (mycotoxin desorption values were less than 14%). The organic solvent (methanol) extracted low percentages of AFB1 (23%) and FB1 (4%), while percentages equal to 71% of ZEA and 90% of OTA were extracted. The chemisorption index decreased in the order FB1 (0.94) > AFB1 (0.74) > ZEA (0.29) > OTA (0.10). This result suggested that the binding of FB1 and AFB1 to MMDA may be stronger than the binding of ZEA and OTA.

Finally, MMDA was analyzed by the equilibrium adsorption isotherm method to study the effect of toxin concentration on the extent of mycotoxin adsorption, and to calculate the maximum capacity and affinity parameters (see for details the experimental part, Example 5). Due to the high efficiency of MMDA in the mycotoxin adsorption, extremely low dosages of smectite of the invention (0.05-0.005%) were used. The adsorption parameters, *i.e.* the maximum adsorption capacity ($C_{max}$) and affinity were calculated by non-linear interpolation and analysis of the experimental adsorption data using the Langmuir and Freundlich mathematical models.

The results of this experiment demonstrate that the bio-organoclay object of the invention had high maximum capacity and affinity values in a physiological pH range. Specifically, for each toxin, the maximum adsorption capacity values expressed as $\mu$g of adsorbed toxin per mg of smectite were in the range between 30-150 $\mu$g/mg for AFB1, 20-150 $\mu$g/mg for FB1, 20-60 $\mu$g/mg for ZEA and 4-20 $\mu$g/mg for OTA. Furthermore, for most toxins, the affinity values ($k_d$) were very high, exceeding 1 mL/$\mu$g. $K_d$ values higher than unity indicate, in fact, high capacity and aptitude of the adsorbent to sequester target toxins even at low concentrations.

The results of these studies and the $C_{max}$ and $K_d$ values determined with the isotherm method suggested that AFB1, FB1 and ZEA bound tightly to the surface of the smectite object of the invention and did not dissociate easily; while OTA adsorption isotherms indicated weaker binding for this toxin. Consequently, the maximum values of adsorption capacity and affinity determined for AFB1, FB1 and ZEA were found to be higher than those measured for OTA, regardless of the pH of the medium.

Mycotoxin adsorption parameters were affected by the pH of the medium differently depending on the toxin.

The adsorptions of AFB1 and ZEA were favored by acidic pH, while the adsorption of FB1 and OTA mainly occurred at neutral pH. These results suggested that the mycotoxin adsorption occurred with different mechanisms depending on the toxin, and that the adsorption properties of the smectite of the invention are different from those of the raw starting clay. In fact, the raw clay (native Na-smectite) adsorbed FB1 and OTA preferably at acidic pH, while their adsorption was negligible or non-existent at neutral pH.

Advantageously, the product of the invention, *i.e.* Na-smectite chemically activated with an inorganic acid and modified with a quaternary ammonium salt of formula $[(CH_3)_3N^+(CH_2)_2OH]\,X^-$, where $X^-$ is a monovalent anion, preferably choline chloride, had polarity of the mineral surface and water occupation in the interlayer such as to facilitate the incorporation of mycotoxin molecules into the smectite interlayer. Since mycotoxins are only moderately soluble in water due to their high $K_{ow}$ (octanol/water partition coefficient) value, this change in polarity resulting from the process of the invention could have facilitated mycotoxin adsorption by the bio-organoclay.

The inventors also studied the effect of temperature on the adsorption process of mycotoxins by the smectite of the invention, and the thermodynamic parameters for the adsorption of AFB1, ZEA, OTA and FB1 were calculated. As illustrated in Example 5 in the experimental part, adsorption isotherms were carried out by testing a fixed amount of bio-organoclay (0.05-0.005%) with buffered solutions of mycotoxins at different concentrations ($\mu$g/mL), at pH=7 and at different temperatures (5, 37, 50 and 70°C). These isotherms allowed us to study the influence of temperature variation on mycotoxin adsorption, and to calculate the thermodynamic parameters ($\Delta$G°, $\Delta$H° and $\Delta$S°) relating to the process.

In studying the adsorption mechanism, both energy and entropy factors were considered to determine which processes occur spontaneously. The measure of the Gibbs free energy change, $\Delta G^0$ (kJ/mol), was used as the fundamental criterion of spontaneity. Reactions occur spontaneously at a given temperature if $\Delta G^0$ is a negative value. The thermodynamic parameters ($\Delta H^0$ and $\Delta S^0$) were calculated using the Van't Hoff equation. The study results showed that enthalpy and entropy values were independent of temperature and that the adsorption process showed a heat capacity ($\Delta$Cp) close to zero. In particular, the $\Delta H^0$ and $\Delta S^0$ values were calculated from the slope and intercept of the lnK versus 1/T plots. The graphs obtained for all mycotoxins at four different temperatures showed good linearity (verified through statistical tests on goodness of fit), also showing values of $R^2 \geq 0.985$. For all mycotoxins, $\Delta G^0$ values were negative at each temperature tested. The result obtained was indicative of a spontaneous, physical adsorption process, with a high affinity of the mycotoxins for the adsorbent surface.

Physisorption involves weak associations including van der Waals, dipole-dipole, induced dipole, and hydrogen bonding. Chemisorption involves a chemical reaction or electron sharing between adsorbent and adsorbate. Physisorption is described as having an enthalpy of $\approx$20 kJ/mol, while chemisorption is generally $\approx$200 kJ/mol. According to the performed test, the enthalpy values calculated for AFB1, ZEA, FB1 and OTA were of -11.05, -21.75, -95.79 and -4.201 kJ/mol, respectively, indicating a predominant physical adsorption phenomenon. In conclusion, the thermodynamic parameters calculated for the adsorption of mycotoxins by the new organophilic smectite provided information on the type of interactions involved in the process.

Hydrophobic interactions could be associated with the adsorption of AFB1, OTA and ZEA (small positive $\Delta H^0$ and $\Delta S^0$ values). Electrostatic interactions explained the higher $\Delta H^0$ and negative $\Delta S^0$ values (-0.234 kJ/(mol*°K) calculated for FB1 adsorption.

According to the present invention, and on the basis of the experimental tests described, the smectite of the invention acts as an adsorbent capable of sequestering, with high capacity and affinity, the main mycotoxins present in products intended for human and animal nutrition, removing them from gastro-intestinal absorption, facilitating their elimination in the feces and preventing toxic effects and acute and chronic intoxications, known as mycotoxicosis, including cancer.

The invention, therefore, also concerns a food product, a supplement or an animal feed including the smectite of the invention as an additive.

The additive at dosages preferably equal to or higher than 0.1% w/v is able to sequester more than 95% of AFB1, FB1, OTA and ZEA (1 $\mu$g/mL), in a wide range of pH values (3-9).

Mycotoxin desorption and isothermal equilibrium assays showed that bio-organoclay can bind mycotoxins stably and with high capacity and affinity.

The final product can be considered safe, as it is obtained using agents listed in the European Union Register of Feed Additives (EC Regulation, n.1831/2003) and food supplements and/or pharmaceuticals.

To verify its efficacy and safety of use, the product of the invention was also tested *in vivo* on laboratory and zootechnical animals and was found to be an excellent multi-mycotoxin decontaminating agent. The smectite of the invention has in fact significantly reduced the values of mycotoxin exposure biomarkers, in particular the values of urinary excretion and oral absorption of mycotoxins, in rats and pigs (Example 6).

The bio-organic clay of the invention has therefore shown high efficacy in the adsorption of AFB1, ZEA, OTA and FB1 in several *in vitro* tests using stringent experimental conditions (Examples 2-5). Using the biomarker approach, the mycotoxin adsorption efficacy of bio-organic clay was confirmed in rats and for all target mycotoxins (Example 6). In pigs, the smectite of the invention was significantly effective for AFB1 and OTA, while it reduced systemic exposure to ZEA and FB1 insignificantly (Example 6).

The smectite of the invention, therefore, proved to be effective as an adsorbent material capable of sequestering structurally different mycotoxins, both *in vitro* and *in vivo.*

The organophilic smectite of the invention, in fact, administered orally by addition to food and feed, at dosages equal to or higher than 0.25% w/v with respect to the weight of the food product, significantly reduced the values of the mycotoxin biomarkers.

At these dosages, the use of the organophilic smectite of the invention did not produce toxic side effects and did not interfere with the absorption of micronutrients (including water-soluble vitamins, fat-soluble vitamins, and minerals).

At the dosages indicated above, the organophilic smectite of the invention has proven capable of preventing the toxic effects of mycotoxins (acute and chronic mycotoxicoses) in living organisms (laboratory animals and pigs).

In a further aspect, the invention relates to the use of the organophilic smectite of the invention for the reduction of gastro-intestinal absorption and exposure of humans and animals to mycotoxins, as measured by specific biomarkers (of exposure and/or effect) of mycotoxins.

The invention therefore concerns the choline-functionalized organophilic smectite of the invention for use in the treatment and prevention of mycotoxicosis in humans and animals.

**Experimental part**

**Example 1: Process for the preparation of an organophilic smectite of the invention**

The process for the preparation of the organophilic smectite involved *(step* a) the use of a raw clay belonging to the group of smectites of the sodium type and sedimentary origin, with high swelling capacity in water, containing 70% or more of montmorillonite as main mineral and 20% of moisture.

For acid pre-activation *(step b)*, the raw mineral was treated with concentrated sulfuric acid (96% purity) at an optimal moisture value of the raw mineral of about 25-30%, for a short period of time (5 min) and at room temperature (preferably 25°C). The acid was added at 15% by weight with respect to the raw clay dry weight and uniformly distributed throughout the clay mass by manual or mechanical mixing. After activation with acid, the crude was dried at 60-65°C in a ventilated oven and ground.

For the functionalization with choline chloride *(step c)*, the acid-activated and ground clay was brought to 16% moisture and treated with choline chloride, at the optimal concentration of 65-75 meq/100g with respect to the clay dry weight. After choline chloride addition, the material was kneaded for 10 minutes at moderate speed and mixed occasionally to ensure homogeneity.

Once the treatment with choline chloride was completed, the kneading was subjected to an extrusion process in order to have an extrudate at temperatures equal to or higher than 45°C *(step d)*, dried in a ventilated oven at 65°C, brought to a moisture of about 9% and ground to have a final product with very fine grain size (500 to 200 μm screens).

**Example 2: Multi-mycotoxin adsorption test (with a single concentration of adsorbent and toxins) and preliminary tests for the development of the organophilic smectite of the invention.**

The native and/or activated and/or modified smectite samples listed in Tables 1-7 reported below were tested for their ability to simultaneously bind AFB1, ZEA, OTA and FB1 in buffer solutions simulating gastric and intestinal pH values (pH 3 and 7, respectively). These values were used during the preliminary study for the development of the organophilic smectite of the invention, as parameters to evaluate the yield and efficacy of the different steps of the process for the preparation of the organophilic smectite.

All materials were tested at a concentration of 0.1% w/v (corresponding to a feed inclusion level of 1 kg/ton) with a multi-mycotoxin solution containing 1 μg/mL of each toxin (corresponding to 1 ppm, part per million). Native and/or activated/modified smectite samples were weighed into 4 mL amber glass vials using an analytical balance. An adequate volume of multi-mycotoxin working solution, in buffer (at different pH values), was added to each vial and mixed vigorously for a few seconds, in order to ensure uniform dispersion of the material in the suspension.

The suspension was then incubated under agitation, with an orbital shaker, thermostatically controlled at 37°C (± 0.5°C), at a speed of 250 rpm for 90 minutes (contact time). After the incubation period, the samples were allowed to settle, and 1 mL-volumes of suspension were transferred into Eppendorf tubes and centrifuged for 20 minutes at 14,000 x g and 25°C.

After centrifugation, supernatant samples were appropriately diluted with buffer (when necessary), then filtered with regenerated cellulose filters (0.2 μm) and analyzed for residual mycotoxin content using a High-Ultra Performance Liquid Chromatography (UHPLC) technique with photodiode detectors (PDA), fluorimetric detectors (FLD) and single quadrupole mass spectrometer (MS) for the simultaneous determination of the mycotoxins of interest.

For all adsorption experiments, two sets of controls were set up. The "blank" control was prepared using the working solution of mycotoxins in buffer without adsorbents. This was subjected to the same test procedure and was used to study the stability of mycotoxins in buffer solutions or any possible non-specific adsorption on the surfaces of the reaction tubes. The "negative" control was, instead, a buffer solution containing the mycotoxin-free material, also treated like the test samples and used to study any material components that could interfere with the mycotoxin chromatographic analysis. All experiments, including blank and negative controls, were set up in triplicate.

In all cases, the amount of mycotoxin adsorbed was calculated as the difference between the amount of mycotoxin in the supernatant of the "blank control" tests (in the absence of adsorbent) and the amount measured in the supernatant of the experimental tests with the adsorbents. This amount was then compared to the amount present in the supernatant of the same controls and expressed as a percentage.

A preliminary study for the development of the organophilic smectite object of the invention was carried out. For this study, the type of smectite to be used for the preparation of the additive (*step a*); the type of acid, the concentration and conditions (time and temperature) for activation (*step b*); the type and concentration of the modifying agent for functionalization, and the operating conditions for modification (*step c*) were evaluated.

The measurement of mycotoxin adsorption values determined as described above was used to evaluate the process yield and efficiency, and to select the best experimental conditions for the preparation of organophilic smectite. The experimental conditions that allowed to have a final product with a broad spectrum of action, capable of simultaneously adsorbing the target mycotoxins, and at physiological pH values (both gastric and intestinal) were considered optimal.

As an example, some of the results of this preliminary study are reported below.

To select the type of smectite to be used for the phases of activation with acid (*step b*) and functionalization with at least one modifying agent (*step c*), smectites with different geological origins (hydrothermal or sedimentary) and of calcium or sodium types, were activated with sulfuric acid at 10% by weight with respect to the smectite dry weight, for a short period of time and at a temperature of 25°C. The adsorbent capacity of native and activated mycotoxins was determined by the method described above and expressed as a percentage.

Table 1 shows the adsorption values of mycotoxins (AFB1, ZEA, OTA and FB1) recorded at acidic and neutral pH for two hydrothermal (calcium) smectites and two sedimentary (sodium) smectites, before (native smectites) and after activation with sulfuric acid.

Table 1. Mycotoxin (AFB1, ZEA, OTA and FB1) adsorption values recorded at acidic and neutral pH after activation of native hydrothermal (calcium-type) or sedimentary (sodium-type) smectites with 10% sulfuric acid.

| | Mycotoxin adsorption (%) | | | | | | | |
|---|---|---|---|---|---|---|---|---|
| | AFB$_1$ | | ZEA | | OTA | | FB$_1$ | |
| **Product** | pH 7 | pH 3 | pH 7 | pH 3 | pH 7 | pH 3 | pH 7 | pH 3 |
| **Native Smectite 1 (hydrothermal)** | 95.8 | 98.7 | 2.8 | 8.0 | 2.8 | 32.6 | 6.9 | 93.8 |
| **Activated Smectite 1** | 97.5 | 98.8 | 8.2 | 20.2 | 0..0 | 47.4 | 86.8 | 95.1 |
| **Native Smectite 2 (hydrothermal)** | 97.9 | 99.5 | 6.9 | 19.8 | 1.3 | 59.0 | 3.4 | 98.1 |
| **Actlvated Smectite 2** | 99.5 | 99.5 | 15.4 | 296 | 2.9 | 69.8 | 44.1 | 98.4 |
| **Native Smectite 3 (sedimentary)** | 97.3 | 99.7 | 5.5 | 21.4 | 2.4 | 61.5 | 5.9 | 94.9 |
| **Activated Smectite 3** | **99.6** | **99.8** | **51.1** | **69.9** | **0.4** | **92.8** | **72.5** | **99.6** |
| **Native Smectite 4 (sedimentary)** | 97.3 | 99.9 | 0.0 | 19.9 | 1.9 | 55.0 | 0.0 | 92.3 |
| **Activated Smectite 4** | **100.0** | **99.7** | **54.9** | **73.6** | **0.4** | **93.5** | **67.0** | **99.9** |

The results in Table 1 show that sodium-type smectites, with sedimentary geological origin, are preferable to calcium-type and hydrothermal ones. After activation with acid, in fact, only with sodium-type and sedimentary smectites it was possible to record significantly higher adsorption values of ZEA, OTA and FB1 compared to those measured for native, non-activated smectites (especially at pH=3). For this reason, smectites containing sodium as an exchangeable metal, and of sedimentary geological origin, were selected for the preparation of the organophilic smectite of the invention.

To choose the type of acid to be used for activation (*step b*), organic or inorganic acids were tested.

In summary, Table 2 reports the mycotoxin adsorption results recorded by activating a sodium smectite of sedimentary geological origin with an organic acid (citric acid) and an inorganic acid (sulfuric acid) at a concentration of 10% by weight with respect to the smectite dry weight. All materials (native smectite and activated smectites) were tested (as described above) at a concentration of 0.1% (w/v), at pH 3 and 7, and against a pool of mycotoxins (AFB1, ZEA, OTA and FB1) at a concentration of 1 $\mu$g/mL each.

Table 2. Effect of the acid type (organic or inorganic) on the adsorbent efficacy of smectites.

| Product | Mycotoxin adsorption (%) | | | | | | | | | | | | | | | |
|---|---|---|---|---|---|---|---|---|---|---|---|---|---|---|---|---|
| | $AFB_1$ | | | | OTA | | | | ZEA | | | | $FB_1$ | | | |
| | pH 7 | | pH 3 | | pH 7 | | pH 3 | | pH 7 | | pH 3 | | pH 7 | | pH 3 | |
| | Mean | SD | Mean | SD | Mean | SD | Mean | SD | Mean | SD | Mean | SD | Mean | SD | Mean | SD |
| Native Smectite (control) | 97.3 | 0.0 | 99.9 | 0.1 | 1.9 | 0.8 | 55.0 | 0.7 | 0.0 | 0.0 | 19.9 | 1.4 | 0.0 | 0.0 | 92.3 | 0.5 |
| Smectite activated with citric acid* | 97.5 | 0.3 | 98.9 | 0.1 | 0.0 | 0.0 | 77.3 | 0.8 | 11.8 | 2.9 | 40.6 | 2.2 | 7.6 | 1.2 | 99.4 | 0.3 |
| Smectite activated with sulfuric acid * | 99.4 | 0.2 | 99.9 | 0.1 | 8.5 | 2.8 | 95.6 | 0.2 | 64.6 | 0.9 | 78.1 | 1.0 | 96.7 | 1.3 | 100 | 0.0 |
| Note: For activation, an acid concentration of 10% by weight with respect to the smectite dry weight was used. | | | | | | | | | | | | | | | | |

From the results in Table 2, with the same concentration of acid used for the activation process, it can be seen that the treatment with sulfuric acid was the most effective in increasing the smectite adsorbent power and, in particular, for OTA at acidic pH, while for ZEA and FB1 the increase was observed at both acidic and neutral pH values.

In another preliminary study, the effect of sulfuric acid concentration on the yield of the activation process in terms of mycotoxin adsorption was evaluated. For this study, a sodium smectite of sedimentary geological origin was activated with sulfuric acid at concentrations of 1%, 4%, 10% and 15% by weight with respect to the smectite dry weight. All materials (native smectite and activated smectites) were tested (as described above) at a concentration of 0.1% (w/v), at pH 3 and 7, and against a pool of mycotoxins (AFB1, ZEA, OTA and FB1) at a concentration of 1 $\mu$g/mL each. Table 3 reports the mycotoxin adsorption values obtained by activating native smectite with increasing concentrations of sulfuric acid (from 1% to 15%).

Table 3. Study of the effect of sulfuric acid concentration on the yield of the smectite activation process in terms of mycotoxin adsorption.

| Product | Mycotoxin adsorption (%) | | | | | | | | | | | | | | | |
| --- | --- | --- | --- | --- | --- | --- | --- | --- | --- | --- | --- | --- | --- | --- | --- | --- |
| | $AFB_1$ | | | | OTA | | | | ZEA | | | | $FB_1$ | | | |
| | pH 7 | | pH 3 | | pH 7 | | pH 3 | | pH 7 | | pH 3 | | pH 7 | | pH 3 | |
| | Mean | SD | Mean | SD | Mean | SD | Mean | SD | Mean | SD | Mean | SD | Mean | SD | Mean | SD |
| Native Smectite (control) | 97.3 | 0.0 | 99.9 | 0.1 | 1.9 | 0.8 | 55.0 | 0.7 | 0.0 | 0.0 | 19.9 | 1.4 | 0.0 | 0.0 | 92.3 | 0.5 |
| 1% sulfuric acid | 97.4 | 0.2 | 99.6 | 0.1 | 2.6 | 0.3 | 50.3 | 2.4 | 6.5 | 5.8 | 20.1 | 3.3 | 10.1 | 3.0 | 93.3 | 0.4 |
| 4% sulfuric acid | 98.1 | 0.3 | 98.8 | 0.3 | 1.8 | 1.0 | 84.0 | 0.4 | 13.4 | 0.8 | 45.5 | 1.3 | 7.9 | 2.1 | 99.6 | 0.1 |
| 10% sulfuric acid | 99.4 | 0.2 | 99.9 | 0.1 | 8.5 | 2.8 | 95.6 | 0.2 | 64.6 | 0.9 | 78.1 | 1.0 | 96.7 | 1.3 | 100 | 0.0 |
| 15% sulfuric acid | **100** | 0.0 | **99.8** | 0.1 | **74.2** | 3.5 | **94.1** | 0.3 | **64.6** | 0.7 | **75.0** | 1.1 | **99.6** | 0.0 | **99.7** | 0.1 |

EP 4 400 549 A1

As can be seen from the data in Table 3, the adsorbent power of activated smectite increased significantly as the concentration of sulfuric acid increased, being maximum at a concentration of 15%. Higher acid concentrations (up to 30%) did not produce substantial increases in the smectite adsorbent power and in any case were not chosen as they could cause a structural alteration in the mineral.

A further study concerned the effect of the temperature of the activation process with sulfuric acid on the adsorbent power of the final, activated product. For this study, a sodium smectite of sedimentary geological origin was activated with sulfuric acid at 15% by weight with respect to the smectite dry weight, for a time of about 5 minutes and at a temperature of 25°C and 80°C. For the native smectite and the activated smectites, the ability to adsorb the target mycotoxins was measured in the experimental conditions described above. The adsorption values (expressed as a percentage) are shown in Table 4.

Table 4. Effect of treatment temperature with 15% sulfuric acid on mycotoxin adsorption at different pH values.

| | Mycotoxin adsorption (%) | | | | | | | |
|---|---|---|---|---|---|---|---|---|
| | $AFB_1$ | | ZEA | | OTA | | $FB_1$ | |
| | pH 7 | pH 3 | pH 7 | pH 3 | pH 7 | pH 3 | pH 7 | pH 3 |
| Native Smectite | 98.0 | 99.4 | 0.0 | 34.0 | 2.1 | 71.3 | 9.3 | 98.0 |
| 15% sulfuric acid - 5 min (80 °C) | 85.7 | 98.6 | 16.8 | 73.7 | 4.3 | 78.9 | 21.6 | 100.0 |
| 15% - sulfuric acid 2 - 5 min (25 °C) | **96.3** | **97.2** | **82.5** | **64.3** | **91.6** | **91.6** | **97.7** | **99.6** |

The results in Table 4 show that, when carried out at 25°C, step b) allowed the mycotoxins to be effectively removed regardless of the pH values of the medium, while higher temperatures (as in in the prior art) gave little or no improvement.

As reported above, in the present invention the acid activation of the smectites was carried out in controlled, not strong conditions, with an acid concentration not exceeding 15% w/w, at low temperature (room temperature) and for a short period of time (5 min), thus not altering the crystalline structure of the starting raw smectite. The results of spectroscopic analyses, including X-ray diffractometry, XRD (Figure 3) and mineralogical analysis (Table 5) demonstrate, in fact, that raw and acid activate smectites have similar mineral structure and comparable chemical-physical properties.

The X-ray diffraction (XRD) technique was used for structural characterization of the materials. Figure 3A shows the diffractogram of a mineral based on sodium smectite with the main peaks indicated. Figure 3B represents the diffractogram of a similar smectite treated as in the invention with 15% w/w of sulfuric acid. The comparison of the XRD spectra of raw and acid-activated smectites highlight retention of the typical smectite peaks even after acid treatment, confirming that acid activation did not substantially alter the structure thereof. The main mineral identified in each of the two samples, in fact, was found to belong to the group of smectites with a dioctahedral structure (montmorillonite), as suggested by the XRD peak at 1,493-1,500 Å ($d_{060}$ value) (Table 5).

Table 5. Values of chemical-physical parameters measured for raw smectite and smectite activated with 15% sulfuric acid.

| Chemical-physical parameters | Measurement Unit | Raw Smectite | Smectite activated with acid |
|---|---|---|---|
| Total free moisture | % | 6.7 | 6.8 |
| pH (5% deionized water dispersion) | | 8.9 | 2.6 |
| Dry residue over 45 micron sieve | % | 26.6 | 31.0 |
| Dry residue over 75 micron sieve | % | 5.2 | 9.1 |
| Swell index | ml/2g | 20 | 9 |
| Methylene blue absorption (CEC/MBA) | mg/g | 250 | 166 |
| Carbonate content | % | 4.6 | 0.5 |
| $Na_2O$ | % | 1.76 | 1.66 |
| MgO | % | 1.76 | 1.53 |

(continued)

| Chemical-physical parameters | Measurement Unit | Raw Smectite | Smectite activated with acid |
|---|---|---|---|
| $Al_2O_3$ | % | 17.94 | 15.78 |
| $SiO_2$ | % | 62.77 | 52.96 |
| $P_2O_5$ | % | 0.11 | 0.14 |
| $K_2O$ | % | 1.22 | 1.05 |
| CaO | % | 3.31 | 3.16 |
| $TiO_2$ | % | 0.35 | 0.33 |
| MnO | % | 0.14 | 0.16 |
| $Fe_2O_3$ | % | 3.98 | 3.73 |
| Smectite | | Dominant | Dominant |
| $d_{001}$ | Å | 12.95 | 13.01 |
| $d_{060}$ | Å | 1.501 | 1.500 |
| Minerals presence | | Opal/cristobalite, quartz, feldspars, calcite | Gypsum, opal/cristobalite |
| Loss on ignition at 960 °C | % | 6.66 | 19.5 |

In a further preliminary study for the development of the organophilic smectite of the invention, the type of modifying agent to be used in step c) for preparing a bio-organoclay with a large adsorption capacity for the mycotoxins of interest was analyzed.

For this study, a sodium smectite of sedimentary geological origin (not activated) was modified with the organic agents listed in Table 6. The choice of these agents was dictated by the need to test non-toxic compounds, capable of exchanging or intercalating in the interlayer of native smectite. All modifiers were tested under the same experimental conditions. Native and functionalized smectites produced by this study were analyzed for their adsorbent power as described above. The adsorption values (expressed as a percentage) are shown in Table 6.

Table 6. Multi-mycotoxin adsorption of a smectite functionalized with different organic modifying agents.

| Product | Mycotoxin adsorption (%) | | | | | | | |
|---|---|---|---|---|---|---|---|---|
| | $AFB_1$ | | ZEA | | OTA | | $FB_1$ | |
| | pH 7 | pH 3 | pH 7 | pH 3 | pH 7 | pH 3 | pH 7 | pH 3 |
| **Native Smectite (control)** | 97.3 | 99.7 | 5.5 | 21.4 | 2.4 | 61.5 | 5.9 | 94.9 |
| **Choline chloride** | **98.6** | **99.0** | **20.8** | **64.9** | **8.2** | **81.6** | **22.1** | **96.6** |
| **Citrulline** | 88.8 | 95.4 | 0.0 | 30.8 | 0.0 | 80.4 | 0.0 | 97.2 |
| **Betaine** | 62.5 | 94.7 | 1.7 | 26.5 | 3.8 | 72.3 | 1.3 | 95.8 |
| **Lysine** | 96.4 | 100.0 | 3.1 | 21.5 | 0.2 | nd | 15.9 | nd |
| **Tyrosine** | 93.5 | 91.1 | 0.4 | 24.8 | 1.0 | 64.5 | 2.6 | 96.4 |
| **PVP 70:30** | 97.8 | 99.6 | 15.3 | 22.1 | 1.9 | 70.1 | 0.0 | 94.7 |
| **PVP 40:60** | 97.6 | 99.5 | 27.5 | 34.1 | 2.0 | 72.0 | 3.0 | 94.1 |
| **Tween 20** | 91.0 | 97.6 | 0.6 | 13.9 | 2.7 | 14.3 | 2.9 | 37.3 |
| **Tween 40** | 90.7 | 97.3 | 2.5 | 16.8 | 2.1 | 14.8 | 3.5 | 43.2 |
| **Tween 60** | 91.7 | 97.2 | 1.7 | 12.8 | 2.9 | 14.5 | 2.9 | 48.2 |
| **Tween 80** | 94.2 | 97.2 | 7.3 | 13.2 | 3.0 | 16.7 | 3.6 | 51.9 |
| **PEG 5 meq** | 12.9 | 23.8 | 5.1 | 1.3 | 2.7 | 1.1 | 3.5 | 2.0 |
| **Carnitine** | 90.8 | 97.5 | n.d. | 21.3 | n.d. | 27.2 | 0.2 | 93.6 |
| **Chitosan** | 97.4 | 100.0 | 0.0 | 14.0 | 5.3 | 51.6 | 4.9 | 92.0 |
| **Pectin** | 95.4 | 98.2 | 0.0 | 12.3 | 4.1 | 48.3 | 4.3 | 88.8 |
| **Lecithin** | 96.2 | 100.0 | 13.8 | 27.6 | 3.0 | 26.3 | 2.5 | 93.1 |

The results in Table 5 led to the conclusion that, among the modifying agents tested in the preliminary study for the development of a bio-organoclay with broad adsorbent power, choline chloride is the most suitable modifying agent for preparing a broad-spectrum product. At pH=3, in fact, the bio-organoclay obtained by treating native smectite with choline chloride showed significantly higher adsorption values for all mycotoxins object of the study. Unlike what was reported in the prior art, lecithin was not found to be ameliorative.

The preliminary study for the development of the bio-organoclay further concerned the effect of the concentration of the optimal modifying agent of step c), choline chloride, on process yield and performances of the intermediate products in terms of the mycotoxin percentage adsorption.

For this study, a sodium smectite of sedimentary geological origin (also in this case not activated) was treated with increasing concentrations (in the range 30-130 meq/100g) of choline chloride at a temperature of 25°C. The results of this study are reported in Table 6 and are expressed as mycotoxin adsorption.

Table 7. Study of the effect of choline chloride concentration (expressed as meq/100g) on the mycotoxin adsorption efficacy.

| Product | Mycotoxin adsorption (%) | | | | | | | |
|---|---|---|---|---|---|---|---|---|
| | AFB$_1$ | | ZEA | | OTA | | FB$_1$ | |
| | pH 7 | pH 3 | pH 7 | pH 3 | pH 7 | pH 3 | pH 7 | pH 3 |
| Native Smectite (control) | 97.3 | 99.7 | 4.3 | 11.8 | 2.0 | 65.2 | 0.0 | 94.2 |
| Choline chloride, 33 | 99.5 | 99.7 | 2.4 | 42.1 | 2.5 | 70.8 | 4.8 | 96.2 |
| Choline chloride, 43 | 97.4 | 99.6 | 0.0 | 23.8 | 3.0 | 50.8 | 0.0 | 89.7 |
| Choline chloride, 55 | 99.0 | 99.6 | 0.0 | 31.4 | 3.6 | 55.7 | 3.3 | 89.2 |
| Choline chloride, 60 | 99.1 | 98.5 | 14.0 | 42.2 | 8.1 | 61.1 | 24.9 | 87.9 |
| Choline chloride, 65 | **98.6** | **99.0** | **20.8** | **64.9** | **8.2** | **81.6** | **22.1** | **96.6** |
| Choline chloride, 79 | 97.1 | 92.3 | 7.9 | 49.0 | 3.2 | 46.9 | 14.9 | 97.3 |
| Choline chloride, 109 | 99.5 | 96.8 | 7.0 | 49.4 | 3.0 | 39.7 | 16.5 | 97.1 |
| Choline chloride, 123 | 98.9 | 98.8 | 3.3 | 45.5 | 2.2 | 34.0 | 3.1 | 96.6 |
| Choline chloride, 124 | 99.4 | 98.1 | 3.5 | 45.5 | 2.2 | 38.0 | 5.7 | 95.5 |

The values in Table 7 show that the mycotoxin adsorption efficacy of bio-organoclays obtained by structural modification of a sodium smectite of sedimentary origin with choline chloride is significantly influenced by the concentration of the modifying agent and has a maximum at a concentration of 65 meq/100g. An increase in adsorption values was recorded especially for those mycotoxins for which native smectite showed poor efficacy at gastric pH.

Finally, a comparison was made between the adsorption capacities of native smectites, those of step b) and those of step c), either subjected to the previous step b) or not subjected to it.

In particular, sodium smectites of sedimentary geological origin were used and treated in the optimal conditions of step b), *i.e.* using sulfuric acid at 15% by weight with respect to the clay dry weight and at a temperature of 25°C. Step c) was also carried out according to the optimal conditions indicated above, i.e. choline chloride was used at a concentration in the range from 65 to 75 meq/100g.

Mycotoxin adsorption values were recorded at neutral and acidic pH using the multi-mycotoxin adsorption and analysis (UHPLC-PDA/FLD/MS) technique described above. The results are reported in Table 8 below.

Table 8: Evaluation of the effect of chemical pre-activation on the adsorption of mycotoxins by bio-organoclays. The organophilic bio-clays were obtained using the optimal concentration of choline chloride (65-75 meq/100g).

| | AFB$_1$ adsorption (%) | | | | ZEA adsorption (%) | | | | OTA adsorption (%) | | | | FB$_1$ adsorption (%) | | | |
|---|---|---|---|---|---|---|---|---|---|---|---|---|---|---|---|---|
| **MMDA** | pH 7 | | pH 3 | | pH 7 | | pH 3 | | pH 7 | | pH 3 | | pH 7 | | pH 3 | |
| | mean | SD | mean | SD | mean | SD | mean | SD | mean | SD | mean | SD | mean | SD | mean | SD |
| **Native Smectite** (*control*) | **95.2** | 1.0 | **96.2** | 1.0 | **2.9** | 0.5 | **35.1** | 3.8 | **0.0** | 0.0 | **64.0** | 2.2 | **8.1** | 1.0 | **95.6** | 0.5 |
| **Activated Smectite** | **96.3** | 0.6 | **97.2** | 0.3 | **82.5** | 0.5 | **64.3** | 0.5 | **91.6** | 0.1 | **91.6** | 0.6 | **97.7** | 0.4 | **99.6** | 0.2 |
| **Bio-organic clay (*no activation step b*)** | **97.5** | 1.2 | **100. 0** | 0.0 | **0.0** | 0.0 | **89.6** | 0.5 | **1.9** | 0.5 | **95.7** | 0.2 | **20.8** | 2.8 | **100. 0** | 0.0 |
| **Bio-organic clay (*activation step b*)** | **98.1** | 1.6 | **99.2** | 1.3 | **82.2** | 1.9 | **80.2** | 2.2 | **85.0** | 2.5 | **84.6** | 2.4 | **98.6** | 0.3 | **97.8** | 0.3 |

As shown in Table 8, the chemical activation of step b) of the invention followed by functionalization with choline chloride made it possible to obtain a product capable of effectively and multifunctionally adsorbing against various toxins, not only at gastric pH (pH=3) but also at intestinal pH (pH=7).

Without being tied to any theory, the inventors believe that the functionalization of activated smectite with the choline salt involves the occurrence of ion exchange reactions between the clay solvated ions and the choline molecules. Following these reactions, the clay lamellae will be intercalated with a lamellar interlayer largely consisting of choline, linked via Coulomb-type interactions with the lamella and the free organic portion in the interlayer. The effect of this exchange resulted in an increase in the interlamellar space and in the $d_{001}$ value of the bio-organoclay.

The chemical-physical and spectroscopic analyses (XRD, XRF, FT-IR, NMR, SEM) also made it possible to confirm the structural modification and change in properties of the final product of the invention with respect to the raw smectite. In particular, swelling index (a test that evaluates the swelling capacity of bentonite under zero-normal-stress); cation exchange capacity value using methylene blue (MBI/CEC) (to measure the degree of bentonite in the mineral); moisture content; pH; carbonate content; loss on combustion (LOI) at 960°C; particle size by laser diffraction were measured. The bentonite samples were characterized by X-ray fluorescence (XRF) analysis to determine the amount of various metals and by X-ray diffraction (XRD) analysis to determine the content of crystalline minerals. Furthermore, further NMR, FT-IR and SEM analyses were carried out to characterize the bio-organoclay and distinguish it from similar compounds.

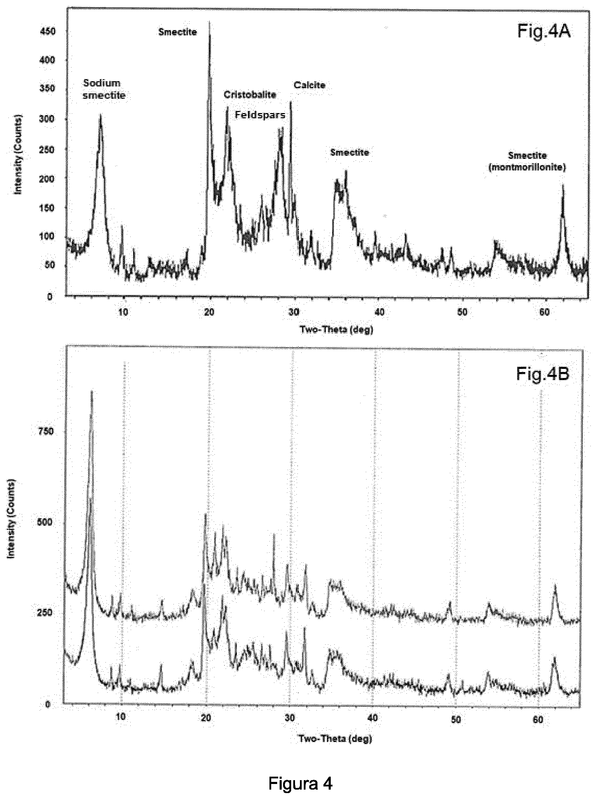

Physical-chemical properties and diffractograms of the raw smectite and of the smectite activated and functionalized with choline, the object of the invention, are shown in Table 9 and Figure 4.

As can be seen from the results in Table 9, bio-organophilic smectite differs from raw smectite in that it does not disperse in water and therefore does not allow the measurement of pH and CEC values. As it does not swell in water, it has a zero swelling index. In bio-organoclay, smectite is still the prevalent mineral, but unlike raw smectite this has higher $d_{001}$ values, being a smectite intercalated with choline, which causes an increase in the interlamellar space. Compared to raw smectite, bio-organoclay has higher values of loss upon calcination determined by the greater presence of organic material in the final product.

The XRD analysis (Figure 4) allowed us to confirm the successful functionalization with intercalation of choline within the silicate lamellae and to have a quantification of the interlayer distance. The diffractograms of the organo-modified smectites (Fig.4B) showed two substantial differences compared to that of the starting sodium smectite (Fig.4A): the diffraction peaks moved to smaller 2Θ angle values and were more defined. Both factors indicate an increase in interlamellar distance and the effective intercalation of the organo-modifier.

Table 9. Chemical-physical properties of a raw sodium-type smectite and of the same smectite activated with sulfuric acid and modified with choline chloride to obtain the bio-organophilic smectite object of the invention.

| Chemical-physical parameters | Measurement Unit | Natural Smectite | Bio-organophilic Smectite |
|---|---|---|---|
| Total free moisture | % | 6.7 | 5.9 |
| pH (5% deionized water dispersion) | | 8.9 | - |
| Dry residue over 45 micron sieve | % | 26.6 | 60.70 |
| Dry residue over 75 micron sieve | % | 5.2 | 35.20 |
| Swell index | ml/2g | 20 | - |
| Methylene blue absorption (CEC/MBA) | mg/g | 250 | - |
| Carbonate content | % | 4.6 | 5.0 |
| $Na_2O$ | % | 1.76 | 2.57 |
| MgO | % | 1.76 | 1.58 |
| $Al_2O_3$ | % | 17.94 | 15.42 |
| $SiO_2$ | % | 62.77 | 49.32 |
| $P_2O_5$ | % | 0.11 | 0.16 |

(continued)

| Chemical-physical parameters | Measurement Unit | Natural Smectite | Bio-organophilic Smectite |
|---|---|---|---|
| $K_2O$ | % | 1.22 | 0.95 |
| CaO | % | 3.31 | 3.60 |
| $TiO_2$ | % | 0.35 | 0.32 |
| MnO | % | 0.14 | 0.17 |
| $Fe_2O_3$ | % | 3.98 | 3.78 |
| Smectite | | Dominant | Very present |
| $d_{001}$ | Å | 12.95 | 14.72 |
| $d_{060}$ | Å | 1.501 | 1.496 |
| Mineral presence | | Opal/cristobalite, quartz, feldspars, calcite | Cristobalite, quartz, feldspars, muscovite |
| Loss on ignition at 960 °C | % | 6.66 | 22.13 |

**Example 3: Effect of the dosage of the smectite of the invention on mycotoxin adsorption**

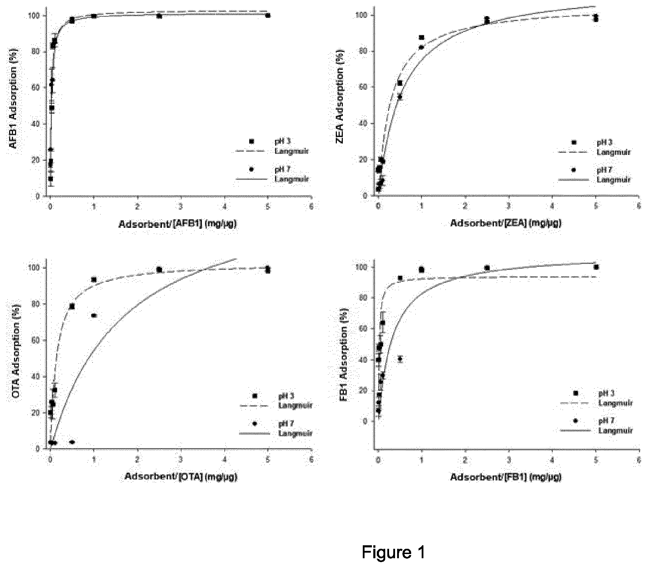

The effect of the dosage of the organophilic smectite (bio-organoclay) of the invention (Example 1) on simultaneous adsorption of mycotoxins was studied using the equilibrium isotherm method. Adsorption experiments were performed in triplicate as described in Example 2, at constant pH (pH 7 and 3), testing a fixed amount of each mycotoxin (AFB1, ZEA, OTA and FB1) at a concentration of 1 $\mu$g/mL, with increasing amounts of adsorbent (0.0005-0.5% w/v corresponding to 0.005-5 mg/mL). The adsorption values measured for each mycotoxin in the pool were expressed as a percentage and plotted as a function of the adsorbent/mycotoxin weight ratio. For all toxins, these values were interpolated using the Langmuir mathematical model. Figure 1 shows the adsorption isotherms obtained at gastric (3) and intestinal (7) pH for mycotoxins of interest. The adsorption values are reported in Table10.

Table 10. Effect of adsorbent dosage on the multi-mycotoxin adsorption efficacy of the bio-organoclay object of the invention.

| Adsorbent dosage (%) w/v | AFB$_1$ adsorption (%) | | | | ZEA adsorption (%) | | | | OTA adsorption (%) | | | | FB$_1$ adsorption (%) | | | |
|---|---|---|---|---|---|---|---|---|---|---|---|---|---|---|---|---|
| | pH 3 | | pH 7 | | pH 3 | | pH 7 | | pH 3 | | pH 7 | | pH 3 | | pH 7 | |
| | mean | SD | mean | SD | mean | SD | mean | SD | mean | SD | mean | SD | mean | SD | mean | SD |
| 0.0005 | 9.8 | 4.1 | 17.3 | 1.1 | 14.2 | 0.7 | 3.2 | 0.7 | 20.1 | 0.9 | 3.5 | 0.0 | 40.0 | 4.2 | 6.9 | 3.4 |
| 0.001 | 19.6 | 6.4 | 25.8 | 0.7 | 13.7 | 0.4 | 3.7 | 1.2 | 20.2 | 3.3 | 3.5 | 0.6 | 48.0 | 2.2 | 12.3 | 3.6 |
| 0.0025 | 48.9 | 2.7 | 61.6 | 8.6 | 15.6 | 0.1 | 6.5 | 0.1 | 25.9 | 0.4 | 3.3 | 0.3 | 48.5 | 0.7 | 17.4 | 0.8 |
| 0.005 | 83.2 | 1.8 | 64.2 | 7.0 | 20.1 | 1.0 | 6.6 | 2.5 | 24.7 | 8.3 | 3.1 | 0.2 | 50.1 | 5.6 | 25.6 | 4.9 |
| 0.01 | 86.5 | 0.0 | 84.7 | 0.1 | 18.9 | 0.3 | 8.4 | 2.7 | 32.4 | 4.0 | 3.1 | 0.1 | 64.1 | 6.7 | 29.9 | 2.3 |
| 0.05 | 96.8 | 0.1 | 98.2 | 0.7 | 62.3 | 1.3 | 54.5 | 1.8 | 78.6 | 1.4 | 3.5 | 0.2 | 93.1 | 0.4 | 40.5 | 1.9 |
| 0.1 | 99.2 | 1.3 | 98.1 | 1.6 | 80.2 | 2.2 | 82.2 | 1.9 | 84.6 | 2.4 | 85.0 | 2.5 | 97.8 | 0.3 | 98.6 | 0.3 |
| 0.25 | 99.6 | 0.5 | 100 | 0.0 | 96.2 | 0.5 | 98.2 | 0.1 | 99.0 | 0.6 | 99.5 | 0.0 | 99.4 | 0.0 | 99.7 | 0.2 |
| 0.5 | 100 | 0.0 | 99.8 | 0.2 | 97.7 | 0.2 | 99.5 | 0.1 | 98.4 | 0.2 | 99.9 | 0.0 | 100 | 0.0 | 99.9 | 0.2 |

As can be seen from Table 10, the adsorption of AFB1, FB1, ZEA, and OTA was significantly affected by the dosage, and the percentage of mycotoxins removed from the buffer solutions at both pH values increased with increasing smectite dosage.

**Example 4: Effect of the pH of the medium on mycotoxin adsorption**

The removal of mycotoxins from an aqueous solution through an adsorption process is, in most cases, highly dependent on the pH of the medium. To study the effect of pH on the simultaneous adsorption of AFB1, ZEA, OTA and FB1 by the smectite prepared in Example 1 (bio-organoclay), independent experiments were performed, in triplicate, at pH values over a very wide range (3-9), using a 0.5% w/v (5 mg/mL) adsorbent dosage and a fixed amount of each mycotoxin (1 $\mu$g/mL).

Adsorption assays and analyses were performed as described in Example 2.

The adsorption data measured for each mycotoxin at different pH values, expressed as a percentage, are shown in Table 11.

Table 11. Effect of the pH of the medium on the multi-mycotoxin adsorption efficacy of the smectite object of the invention (bio-organoclay).

| pH | Adsorption (%) | | | | | | | |
|---|---|---|---|---|---|---|---|---|
| | AFB$_1$ | | ZEA | | OTA | | FB$_1$ | |
| | mean | SD | mean | SD | mean | SD | mean | SD |
| 3 | 100.0 | 0.0 | 100.0 | 0.0 | 99.5 | 0.0 | 99.5 | 0.0 |
| 4 | 99.6 | 0.5 | 99.7 | 0.4 | 98.9 | 1.0 | 99.1 | 0.4 |
| 5 | 99.3 | 0.2 | 99.4 | 0.8 | 95.3 | 0.1 | 99.4 | 0.8 |
| 6 | 99.4 | 0.9 | 100.0 | 0.0 | 99.7 | 0.0 | 99.3 | 0.3 |
| 7 | 100.0 | 0.0 | 100.0 | 0.0 | 99.6 | 0.0 | 99.6 | 0.0 |
| 8 | 100.0 | 0.0 | 100.0 | 0.0 | 99.5 | 0.1 | 99.3 | 0.3 |
| 9 | 100.0 | 0.0 | 100.0 | 0.0 | 100.0 | 0.0 | 99.4 | 0.0 |

Under these experimental conditions, the smectite of the invention sequestered AFB1, ZEA, OTA and FB1 to an equal extent and in all pH ranges, recording adsorption values of $\geq$95% in all cases.

A further study was carried out to evaluate whether a change in the pH of the medium or the treatment (extraction) of the adsorbent pellet with an organic solvent (methanol) is able to produce a release of the mycotoxins sequestered by the bio-organoclay object of the invention.

For this study, 10 mg of bio-organoclay were weighed in a 4 mL screw cap tube and suspended with 2 mL of multi-mycotoxin working solution at pH 3 in citrate buffer, containing 1 $\mu$g/mL of each mycotoxin (AFB1, ZEA, OTA and FB1). As in the previous experiment (pH effect study), the final dosage of adsorbent was 0.5% (w/v). The samples were incubated at 37°C for 90 minutes in a rotary shaker at 250 rpm; after which, they were centrifuged, and the supernatants completely removed and analyzed by UHPLC-FLD/PDA/MS techniques to measure the residual mycotoxin content and to calculate the adsorption values. The adsorbent pellets, containing the mycotoxin/bio-organoclay complex were instead washed with 2 mL of phosphate buffer (pH 7) and stirred for 30 minutes at 250 rpm; then, they were centrifuged and the supernatants analyzed to measure the concentration of mycotoxins released from the pellet and to calculate the relative desorption values. Subsequently, the same method was repeated by resuspending (extracting) the pellet with methanol. Also in this case, the desorption value was determined. Desorption studies were conducted in triplicate. Mycotoxin adsorption at pH=3, desorption at pH=7, and methanol desorption values were calculated for each toxin and expressed as a percentage. Furthermore, the chemisorption index ($C_{\alpha}$, dimensionless quantity), defined as the ratio between the amount of bound toxin ($C_a$) subtracted from the desorbed amount ($C_d$) and compared to the initial amount ($C_i$) (i.e., $C_{\alpha} = (C_a-C_d)/C_i$), was calculated for each toxin,

Table 12 shows the values of adsorption at pH=3, desorption at pH=7, desorption with methanol, total desorption and the chemisorption index measured for the bio-organoclay object of the invention.

Table 12. Mycotoxin desorption values due to changes in the pH of the medium and extraction with methanol.

| Toxin | Adsorption at pH=3 (%) | | Desorption at pH=7 (%) | | Desorption MeOH (%) | | Total Desorption (%) | | Chemisorption | |
|---|---|---|---|---|---|---|---|---|---|---|
| | *mean* | *SD* | *mean* | *SD* | *mean* | *SD* | *mean* | *SD* | *mean* | *SD* |
| AFB$_1$ | 97.3 | 1.1 | 4.1 | 0.9 | 22.8 | 4.5 | 26.0 | 4.1 | 0.74 | 0.04 |
| FB$_1$ | 99.7 | 0.3 | 2.3 | 2.1 | 4.1 | 0.6 | 6.4 | 2.5 | 0.94 | 0.02 |
| ZEA | 99.6 | 0.1 | 0.1 | 0.2 | 71.4 | 0.9 | 71.4 | 5.0 | 0.29 | 0.05 |
| OTA | 99.6 | 0.0 | 14.2 | 1.3 | 88.9 | 12.0 | 90.4 | 10.5 | 0.10 | 0.10 |

In agreement with previous results, mycotoxin adsorption at pH=3 was 100% for all target toxins. A change in the pH of the medium (from acidic to neutral) did not release the adsorbed toxins (mycotoxin desorption values were less than 14%). The organic solvent (methanol) extracted low percentages of AFB1 (23%) and FB1 (4%), while percentages equal to 71% of ZEA and 90% of OTA were extracted. The chemisorption index decreased in the order FB1 (0.94) > AFB1 (0.74) > ZEA (0.29) > OTA (0.10). This result suggested that the binding of FB1 and AFB1 to MMDA may be stronger than the binding of ZEA and OTA.

**Example 5: Equilibrium adsorption isotherms to determine the extent of mycotoxin adsorption (maximum capacity and affinity) and thermodynamic parameters.**

The equilibrium adsorption isotherm method was used to study 1) the mechanism of mycotoxin adsorption by the smectite object of the invention; 2) the effect of toxin concentration and temperature on the adsorption process; 3) to determine the extent of adsorption and the adsorption parameters (maximum capacity and affinity), 4) as well as, to measure the thermodynamic parameters.

For this purpose, two series of adsorption isotherms were performed under different experimental conditions. In all cases, independent experiments were performed in triplicate.

The first set of equilibrium isotherms was carried out at constant temperature (37°C) and pH (pH=3 and pH=7), testing a fixed amount of material, i.e. the product of the invention of Example 1, against the mycotoxins of interest (AFB1, ZEA, OTA and FB1) in buffered solutions and with increasing toxin concentration. In this way it was also possible to study the effect of toxin concentration on the adsorption process. Due to the high adsorption efficiency of the smectite object of the invention, very low dosages of material were used, in the range of 0.05-0.005% w/v.

The amount of mycotoxin adsorbed per unit mass of the test material was calculated using the following equation: $Q_{eq} = [(C_0 - C_{eq}) V] / m$; where $Q_{eq}$ = amount of mycotoxin adsorbed per milligram of material under test (μg/mg); $C_0$ = concentration of mycotoxins in the supernatants of the "blank" control tests without test material (μg/ml); $C_{eq}$ = residual concentration of mycotoxins in the supernatant of the experimental tests with the adsorbent material, at equilibrium (μg/mL); V = volume of solution (mL); m = mass of test material (mg). The adsorption curves (isotherms) were obtained by plotting the amount of mycotoxin adsorbed per unit mass of product ($Q_{eq}$) with respect to the concentration of the toxin in the external phase ($C_{eq}$), under equilibrium conditions, $Q_{eq} = f(C_{eq})$. These values were analyzed using the SigmaPlot program (Systat.com, version 12.0) and interpolated with the Langmuir and Freundlich isotherm equations. The model that fitted the experimental data most accurately was used to describe the system and predict the adsorption behavior, as well as to calculate the maximum adsorption capacity ($C_{max}$) and affinity ($K_d$) parameters. $C_{max}$ (maximum adsorption capacity) values were expressed as the amount of adsorbed mycotoxin (μg) per mg of product. The affinity is correlated with the adsorption energy and was expressed as mL/μg. All adsorption isotherms consisted of 8 or more experimental points.

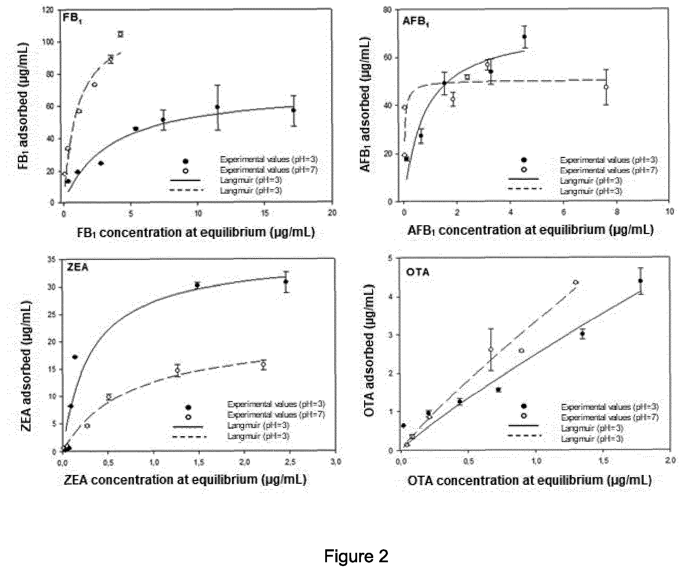

Figure 2 shows the graphs of the equilibrium adsorption isotherms for AFB1, ZEA, OTA and FB1 obtained for the bio-organoclay object of the invention at different pH values (3 and 7).

Table 13 shows the maximum adsorption capacity and affinity values measured for bio-organoclay using the equilibrium isotherm method. This table shows the $C_{max}$ and $K_d$ values calculated for each toxin at different pH values, the mathematical model used for interpolation and analysis of the experimental data, and the dosage of adsorbent material.

Table 13: Equilibrium isotherm parameters calculated at 37°C and different pH values for the adsorption of aflatoxin B1 (AFB1), zearalenone (ZEA), ochratoxin A (OTA) and fumonisin (FB1) by bio-organoclay object of the invention.

| | $AFB_1$ | | $FB_1$ | | ZEA | | OTA | |
|---|---|---|---|---|---|---|---|---|
| | pH 7 | pH 3 | pH 7 | pH 3 | pH 7 | pH 3 | pH 7 | pH 3 |
| Model [1] | Langmuir | Langmuir | Langmuir | Langmuir | Langmuir | Langmuir | Freundlich | Freundlich |
| $C_{max}$ (µg/mg) | 50±3 | 73±8 | 115±8 | 71±6 | 22±1 | 36±3 | 4.3±0.1 | 4.4±0.3 |
| $K_d$ (mL/µg) | 28±8 | 1.3±0.5 | 1.0±0.2 | 0.3±0.1 | 1.4±0.2 | 3.2±1.5 | 3.3±0.1 | 2.5±0.1 |
| dosage [2] (%, w/w) | 0.005 | 0.005 | 0.005 | 0.005 | 0.005 | 0.005 | 0.05 | 0.005 |
| (1) Mathematical model used for interpolation of the adsorption isotherm data and to calculate adsorption parameters; (2) Adsorbent dosage used for equilibrium adsorption isotherms | | | | | | | | |

The results of these studies suggested that AFB1, FB1 and ZEA bound tightly to the surface of the smectite object of the invention and did not dissociate easily, while OTA adsorption isotherms indicated weak binding of this toxin. Consequently, the maximum values of adsorption capacity and affinity determined for AFB1, FB1 and ZEA were found to be higher than those measured for OTA, regardless of the pH of the medium.

Mycotoxin adsorption parameters were affected by the pH of the medium differently, depending on the toxin. The adsorptions of AFB1 and ZEA were favored by acidic pH, while the adsorption of FB1 and OTA mainly occurred at neutral pH. These results suggested that the adsorption of mycotoxins occurred with different mechanisms depending on the toxin and that the adsorption properties of the smectite of the invention are different from those of the raw starting clay. In fact, the raw clay (native Na-smectite) adsorbed FB1 and OTA preferably at acidic pH, while their adsorption was negligible or non-existent at neutral pH.

Advantageously, the product of the invention, i.e. Na-smectite chemically activated and modified with choline chloride, had a polarity of the mineral surface and water occupation in the interlayer such as to facilitate the incorporation of mycotoxin molecules into the intermediate layer of the smectite. Since mycotoxins are only slightly soluble in water based on their high $K_{ow}$ value (octanol/water partition coefficient), this change in polarity could have facilitated the adsorption of mycotoxins by the bio-organoclay.

The second set of equilibrium adsorption isotherms was carried out at pH=7 (constant) and at different temperatures, i.e. $5.0\pm0.5°C$, $37.0\pm0.5°C$, $50.0\pm0.5°C$ and $70.0\pm0.5°C$. These isotherms allowed us to study the influence of temperature variation on mycotoxin adsorption, as well as to calculate the thermodynamic parameters ($\Delta G°$, $\Delta H°$ and $\Delta S°$) relating to the adsorption process. Also in this case, the isotherms were carried out by testing a fixed quantity of bio-organoclay (0.05-0.005% w/v) with buffered solutions of mycotoxins at different concentrations ($\mu g/mL$). The isotherms consisted of 8 or more experimental points.

In adsorption studies, it is necessary to study both energy and entropy factors in order to determine which processes occur spontaneously. The change in Gibbs free energy, $\Delta G^0$ (kJ/mol), is considered the fundamental criterion of the spontaneity of a process. Reactions occur spontaneously at a given temperature if $\Delta G^0$ is a negative value. Gibbs free energy is calculated from the thermodynamic equilibrium constant (apparent equilibrium constant), $K^0$, which is defined as follows:

$$K^0 = a_s/a_e = v_s\, q_e/v_e\ C_e$$

whereas is the activity of the adsorbed mycotoxin, $a_e$ is the activity of the mycotoxin in solution at the equilibrium, $v_s$ is the activity coefficient of the adsorbed mycotoxin and $v_e$ is the activity coefficient of the mycotoxin in solution at the equilibrium. As the concentration of mycotoxins in the solution decreases and approaches zero, the activity coefficient v approaches unity. In this case, the equation may be written as:

$$\lim (a_s/d_e) = q_e/C_e = K^0$$

$$qe \rightarrow 0$$

$q_e$ is the molar concentration of adsorbed mycotoxins (mol/kg); $C_e$ is the residual concentration of toxins at equilibrium (mol/L). The values of $K^0$ are obtained by interpolating $\ln(q_e/C_e)$ with respect to $C_e$ and extrapolating the $C_e$ value at zero. The value of $K^0$ is used in the following equation to determine Gibbs free energy of adsorption ($\Delta G^0$).

$$\Delta G^0 = -RT \ln K^0$$

Enthalpy ($\Delta H^0$, kJ/mol) and entropy ($\Delta S^0$, kJ/mol K) were calculated using the van't Hoff equation:

$$\ln K^0 = -\Delta H^0/RT + \Delta S^0/R$$

R is the universal gas constant (8.314 J mol$^{-1}$ K$^{-1}$); T is the absolute temperature (K). The values of $\Delta H^0$ and $\Delta S^0$ were determined from the slope and intercept of the $\ln K^0$ versus 1/T plots.

Table 14 shows the thermodynamic parameters for the adsorption of AFB1, ZEA, FB1 and OTA measured at different temperatures and at pH=7 for the organophilic smectite object of the invention.

Table 14. Thermodynamic parameters for mycotoxin adsorption measured at different temperatures and at pH=7 for the organophilic smectite object of the invention.

| Thermodynamic parameters | AFB$_1$ | FB$_1$ | ZEA | OTA |
|---|---|---|---|---|
| $\Delta$H$^0$ *(kJ/mol)* | -11.05 | -95.79 | -21.75 | -4.201 |
| $\Delta$S$^0$ *(kJ/mol*°K)* | 0.059 | -0.234 | 0.001 | 0.045 |
| $\Delta$G$^0$ (5°C) *kJ/mol* | -27.33 | -30.70 | -21.93 | -16.77 |
| $\Delta$G$^0$ (37°C) *kJ/mol* | -29.20 | -23.21 | -21.95 | -18.22 |
| $\Delta$G$^0$ (50°C) *kJ/mol* | -29.96 | -20.14 | -27.96 | -18.81 |
| $\Delta$G$^0$ (70°C) *kJ/mol* | -31.13 | -15.49 | -21.97 | -19.71 |

The results of the study showed that enthalpy and entropy values were independent of the temperature and that the mycotoxin adsorption process showed a heat capacity ($\Delta$Cp) close to zero. In particular, the values of $\Delta$H° and $\Delta$S° were calculated from the slope and intercept of the lnK versus 1/T plots. The graphs obtained for all mycotoxins at four different temperatures showed good linearity (verified through statistical tests for goodness of fit), also showing values of $R^2 \geq 0.985$. For all mycotoxins, $\Delta$G$^0$ values were negative at each temperature tested. The result obtained was indicative of a spontaneous, physical adsorption process, with a high affinity of the mycotoxins for the adsorbent surface. Physisorption involves weak associations including van der Waals, dipole-dipole, induced dipole, and hydrogen bonding. Chemisorption involves a chemical reaction or electron sharing between adsorbent and adsorbate. Physisorption is described as having an enthalpy of $\approx$20 kJ/mol, while chemisorption is generally $\approx$200 kJ/mol.

According to the performed test, the enthalpy values calculated for AFB1, ZEA, FB1 and OTA were -11.05, -21.75, -95.79 and -4.201 kJ/mol, respectively, indicating a predominant physical adsorption phenomenon. In conclusion, the thermodynamic parameters calculated for the adsorption of mycotoxins by the new organophilic smectite provided information on the type of interactions involved in the process. Hydrophobic interactions could be associated with the adsorption of AFB1, OTA and ZEA (small positive $\Delta$H$^0$ and $\Delta$S$^0$ values). Electrostatic interactions explained the higher $\Delta$H$^0$ and negative $\Delta$S$^0$ values (-0.234 kJ/(mol*°K) calculated for FB1 adsorption.

**Example 6: Verification of the efficacy of the bio-organoclay object of the invention in reducing the bioavailability of mycotoxins in *in vivo* tests with animals (rats and pigs).**

As required by the European Food Safety Authority (EFSA) guidelines, the organophilic smectite object of the invention with adsorbent activity against a broad spectrum of mycotoxins (AFB1 , ZEA, OTA and FB1) was tested *in vivo,* in laboratory animals (rats) and pigs, using the "biomarkers" approach (exposure biomarkers).

EFSA requires, in fact, that additives to be proposed as mycotoxin decontaminants for feeds are tested, *in vitro* and *in vivo,* to evaluate their mode of action, specificity, efficacy and safety of use. *In vivo* studies in which non-specific parameters are measured (*e.g.* growth, feed conversion, etc.) are not considered sufficient to demonstrate the efficacy of these additives. Toxicokinetic studies, based on the absorption, distribution, metabolization and excretion (ADME) of mycotoxins, are therefore necessary to evaluate the possible effects of the additive on toxin absorption in target organisms. Furthermore, EFSA requires that the interaction of additives with the absorption of dietary micronutrients is studied.

For the aforementioned reasons, the *in vivo* studies reported in the present patent application and performed to evaluate the efficacy of bio-organoclay as a multi-mycotoxin adsorbent were short-term toxicokinetic studies, based on the biomarker approach, and focused on the plasma and/or urinary concentration profiles of AFB1, ZEA, OTA, FB1 and their metabolites in pigs and rats. Specifically, mycotoxins and their metabolites were determined in plasma (for pigs) and urine (for rats) after oral administration of the mycotoxins (as a mixture or individually) with or without bio-organoclay.

The toxicokinetic parameters were compared between the groups, *i.e.* the group of control animals to which only mycotoxins were administered (individually or as a mixture) and the animals of the "additive-treated" group to which mycotoxins were administered in addition to the organophilic smectite object of the invention. The number of animals/replicates for each group allowed comparison and statistical analysis of the results.

The interaction of bio-organoclay with the absorption of micronutrients was also studied in pigs. Vitamin A (VitA), vitamin B12 (Vit B12), and calcium (Ca) (as model compounds for water-soluble vitamins, fat-soluble vitamins, and minerals, respectively) were quantified in serum samples taken before administration and at several timepoints after the bio-organoclay administration.

The animal testing was carried out in collaboration with Bari University (for rat testing) and with Ghent University (Belgium, for pig testing) and after approval by the respective Ethics Committees and the Bodies in Charge of Animal Welfare.

The bio-organoclay object of the invention was administered to rats and pigs, by intragastric bolus, at a concentration corresponding to an inclusion level in the feed of 0.5% (w/w). The differences between studies with rats and pigs are listed below. Mycotoxins were administered individually to rats, while a cocktail of the four toxins was tested with piglets. The doses of mycotoxins administered to individual animals and expressed as mg of toxin per kg of body weight (mg/kg bw) are reported below. *Rats:* 12 mg/kg of FB1, 1 mg/kg of ZEA, 0.5 mg/kg of OTA and 0.12 mg/kg of AFB1. The amount of daily feed intake by the rats was 100 g/kg body weight.

*Pigs:* 2.0 mg/kg of FB1, 0.33 mg/kg of ZEA, 0.10 mg/kg of AFB1, 0.05 mg/kg of OTA. The feed intake was equal to 50 g/kg body weight.

In the tests with pigs, the animals in the treated groups received only one dose of the product of the invention mixed with the mycotoxin cocktail, while the rats were treated with the product of the invention throughout the experiment. In the latter case, a daily dose of clay was suspended in apple juice to make it more palatable, diluted with water and administered *ad libitum*. This approach was suggested to protect animals from those mycotoxins (ZEA, FB1 and OTA) which undergo enterohepatic circulation after intestinal absorption. Furthermore, the effect of the presence of feed on the absorption of mycotoxins was determined only in pigs.

Rats were housed individually in metabolic cages to collect urine samples at different times: from 0h (before administration) up to 72 h after administration of AFB1, ZEA or FB1; and from 0 h up to 336 h for OTA (due to the OTA long half-life). Plasma samples were collected from the pigs, at different timepoints, from 0 to 72 h after administration of the bolus with the toxins +/- bio-organoclay.

The mycotoxin biomarkers analyzed in rat urine were AFM1, ZEA and its phase I biotransformation metabolites ($\alpha$-ZOL, $\beta$-ZOL, and $\beta$-ZAL), OTA, and FB1. The biomarkers of mycotoxin exposure were analyzed using validated UPLC-FLD/PDA/MS methods. Data on normalized urinary excretion of mycotoxins and/or metabolites were expressed as (n)mol of mycotoxin/(n)mol of creatinine. The area under the curve ($AUC_{0 \to t}$) values in the time range 0 - 72/320 hours and the maximum concentration in urine ($C_{max}$) were calculated for the target mycotoxins and/or metabolites and were used to compare the "control" and "treated" groups.

For the pig experimentation, mycotoxin biomarkers were analyzed in pig plasma and were AFB1, OTA, FB1 and ZEA-glucuronide. Biomarker analysis was performed using a validated LC-MS/MS method. Toxicokinetic analysis of plasma concentration/time profiles for individual mycotoxins was performed by non-compartmental analysis and the following parameters were calculated: area under the plasma concentration/time curve from 0 h to the last sampling point with values above LOQ ($AUC_{0 \to t}$) or at infinity ($AUC_{0 \to \infty}$), maximum plasma concentration ($C_{max}$), time to maximum plasma concentration ($T_{max}$), elimination half-life ($T_{1/2\ el}$) and elimination rate constant ($k_{el}$). As in the studies with rats, the effect of the smectite of the invention on ZEA, OTA, FB1 and AFB1 oral absorption was evaluated by comparing the toxicokinetic parameters between the control and bio-organoclay-treated groups.

The results of both *in vivo* experiments with the bio-organoclay object of the invention show that the clay added to contaminated feeds is able to significantly reduce the urinary excretion and oral absorption (plasma concentration) of some of the mycotoxins of greater toxicological and commercial interest (aflatoxins, ochratoxins, zearalenones and fumonisins).

In particular, the efficacy of mycotoxin adsorption by the bio-organoclay of the invention was confirmed in rats for all the mycotoxins tested. In pigs, the bio-organoclay was significantly effective for AFB1 and OTA, while it reduced systemic exposure to ZEA and FB1 non-significantly.

Regarding the interaction of bio-organoclay with micronutrients and the reduction of their absorption, the bio-organoclay did not significantly alter the serum concentrations of Vit A, Vit B12 and Ca at a dose of 0.25 g clay/kg of body weight in pigs.

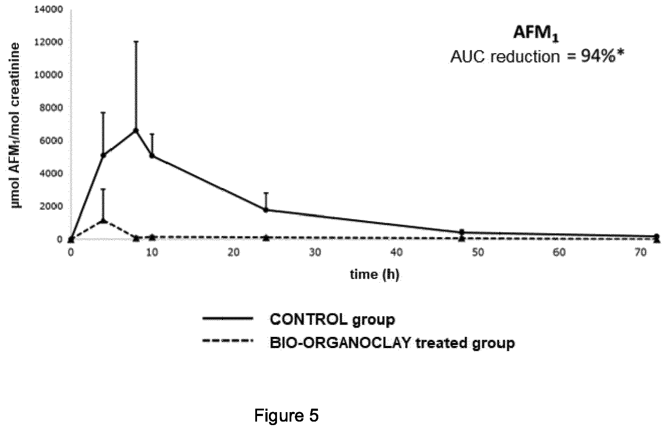

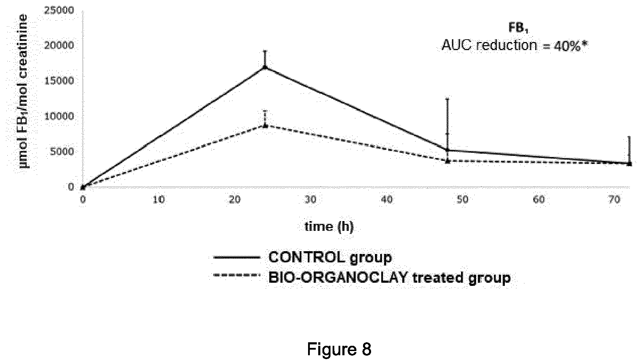

Tables 15 - 21 and Figures 5 - 8 show the urinary excretion values measured for AFB1, ZEA, OTA and FB1 in rats from the "control" and "treated" groups with bio-organoclay.

Table 15. AFM1 urinary excretion in rats treated with 30 $\mu$g of AFB1 and 125 mg of bio-organoclay. The animals in the control group received the toxin in the absence of the clay. Results are reported as mean $\pm$ SD (n=6).

| Time (h) | Aflatoxins concentration in rat urine ($\mu$mol AFM$_1$/mol creatinine) | |
| --- | --- | --- |
| | Control | Bio-organoclay |
| 4 | 5095$\pm$2607 | 1175$\pm$1869 |
| 8 | 6607$\pm$5431 | 81$\pm$85 |
| 10 | 5088$\pm$1320 | 136$\pm$83 |

(continued)

| Time (h) | Control | Bio-organoclay |
|---|---|---|
| | Aflatoxins concentration in rat urine ($\mu$mol AFM$_1$/mol creatinine) | |
| 24 | 1770±1034 | 113±36 |
| 48 | 408±150 | 68±54 |
| 72 | 179±99 | 0.1±0.1 |

Table 16. Effect of bio-organoclay in reducing AFM1 urinary excretion in rats treated with 30 $\mu$g of AFB1 and 125 mg of the product. Results are reported as median (n=6).

| | AUC$_{0\to72}$ (h*$\mu$mol AFM$_1$/mol creatinine) | C$_{max}$ (8h) ($\mu$mol AFM$_1$/mol creatinine) |
|---|---|---|
| Control | 107242 | 5873 |
| Bio-Organoclay | 6219 | 84 |
| | Mann-Whitney rank sum test (P=0.002) | Mann-Whitney rank sum test (P=0.016) |

As can be seen from the data reported in Tables 15 and 16 and in Figure 5, the bio-organoclay administered by gastric gavage to rats, at a dose of 125 mg/animal, was effective in sequestering a single oral dose of 30 $\mu$g AFB1/animal. The bio-organoclay has, in fact, reduced by 90% the toxin excretion parameters (AUC$_{0\to t}$ and C$_{max}$) and the overall total excretion of AFM1. The total excretion of AFM1 calculated taking into account the amount of toxin measured in urine samples collected up to 72 hours corresponded to 0.3±0.1% of the administered dose. The control animals excreted a tenfold greater amount of toxin (3.1±1.5%), as they were not protected by the bio-organoclay. These results are in line with the *in vitro* data showing that a weight ratio between AFB1 and bio-organoclay, equal to 1:1000 (or higher) is able to reduce the toxin adsorbable fraction by 90%. In the present study, the ratio of AFB1 to binder was 1:4000.

Table 17. Effect of the bio-organoclay in reducing urinary excretion parameters of ZEA (and phase I metabolites) in rats treated with 300 $\mu$g of ZEA and 125 mg of clay. Results are reported as mean ± SD or medians (n = 9). The results obtained for the group treated with bio-clay were compared with those of the control group for a significant difference.

| ZEA+MET | | AUC$_{0\to72}$ (mean±SD) h*nmol ZEA+MET/nmol creatinine | C$_{max}$ (median) nmol ZEA+MET/nmol creatinine |
|---|---|---|---|
| Control | | 672±176 | 48 |
| Bio-Organoclay | | 594±205 | 17 |
| | | t-test (P=0.407) | Mann-Whitney rank sum test (P=0.019) |

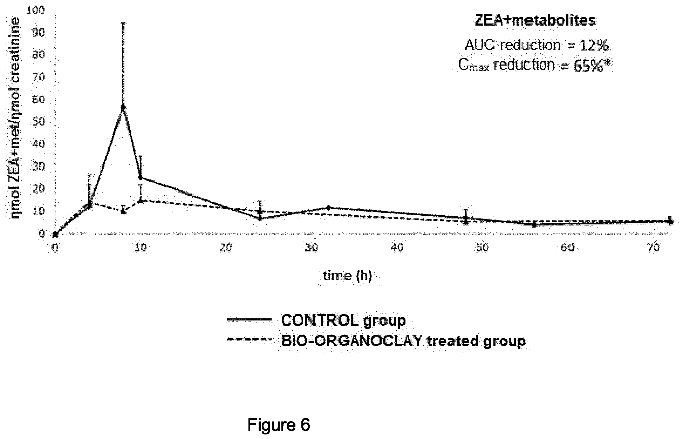

As reported in Table 17 and Figure 6, the bio-organoclay-based product object of the invention significantly reduced the overall excretion of ZEA+metabolites at T$_{max}$ (8h) with respect to the control group. In particular, the bio-organoclay reduced the concentration of ZEA+metabolites excreted at the time of maximum excretion (8h) by 65% and significantly (p=0.019). The overall excretion of ZEA and metabolites was slightly affected by bio-organoclay. Lower AUC values with respect to control were determined for the main urinary biomarkers of ZEA ingestion, *i.e.* ZEA and $\alpha$-ZOL.

Table 18. Urinary excretion of OTA in rats treated with 125 $\mu$g of OTA and 125 mg of bio-organoclay. The animals in the control group received the toxin in the absence of the clay. Results are reported as mean ± SD (n=6).

| Time (h) | Control | Bio-organoclay |
|---|---|---|
| | OTA concentration in rat urine ($\mu$mol OTA/mol creatinine) | |
| 4 | 5113±4731 | 3448±2419 |
| 8 | 5459±2340 | 2688±1056 |

(continued)

| Time (h) | OTA concentration in rat urine (μmol OTA/mol creatinine) | |
| --- | --- | --- |
| | Control | Bio-organoclay |
| 10 | 6718±3776 | 1738±467 |
| 24 | 4452±1745 | 2343±1252 |
| 48 | 3449±2918 | 2679±1517 |
| 72 | 4046±2388 | 1572±418 |
| 296 | 3689±1692 | 1528±1360 |
| 312 | 361±257 | 342±60 |
| 320 | 425±135 | 549±228 |
| 336 | 1054±1660 | 428±174 |

Table 19. Effect of the bio-organoclay in reducing OTA urinary excretion in rats treated with 125 μg of OTA and 125 mg of the product. Results are reported as mean ± SD (n=6).

| | $AUC_{0 \to 336}$ (h*μmol OTA/mol creatinine) | $C_{max}$ (10h) (μmol OTA/mol creatinine) |
| --- | --- | --- |
| Control | 996356±329242 | 6718±3776 |
| Bio-organoclay | 456273±191549 | 1738±467 |
| | t-test (P=0.011) | t-test (P=0.077) |

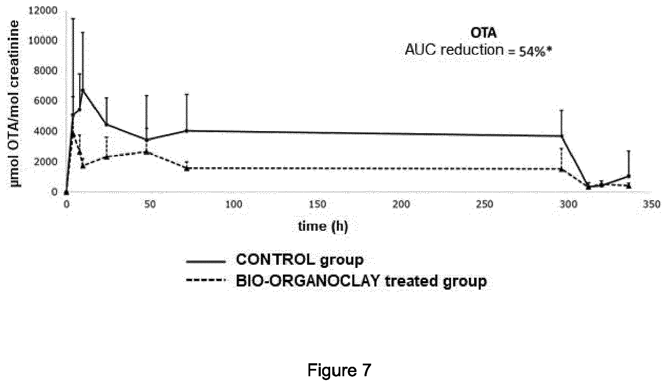

From the results in Table 18 and 19 and Figure 7, it can be concluded that the bio-organoclay was effective in reducing the absorption of OTA in rats. Urinary excretion of OTA in the animals of the bio-organoclay treated group was about 54% lower than in the animals of the control group. A significant reduction in urine toxin concentration was recorded at all sampling timepoints. This effect was observed mainly at the timepoints of toxin maximum absorption (up to 10h). The toxicokinetic parameters of urinary OTA excretion measured for animals in the treated group were significantly lower than those calculated for animals in the control group (p<0.05). The total amount of OTA excreted by the treated rats (1.2±0.5%) was significantly lower than the amount of OTA excreted by the control rats (2.3±0.7%).

Table 20. FB1 urinary excretion in rats treated with 3 mg of FB1 and 125 mg of bio-organoclay. The animals in the control group received the toxin in the absence of the clay. Results are reported as mean ± SD (n=6).

| Time (h) | $FB_1$ concentration in rat urine (pmol FB1/mol creatinine) | |
| --- | --- | --- |
| | Control | Bio-organoclay |
| 24 | 16895±2337 | 8730±2073 |
| 48 | 5260±7183 | 3725±3775 |
| 72 | 3311±3775 | 3335±1199 |

Table 21. Effect of bio-organoclay in reducing FB1 urinary excretion in rats treated with 3 mg of FB1 and 125 mg of product. Results are reported as mean ± SD (n=6).

| | $AUC_{0 \to 72}$ (h*μmol $FB_1$/mol creatinine) | $C_{max}$ (24h) (μmol $FB_1$/mol creatinine) |
| --- | --- | --- |
| Control | 551032±169223 | 16045±2337 |
| Bio-organoclay | 328061±120743 | 9155±1649 |
| | t-test (P=0.025) | t-test (P=0.001) |

In humans and animals, fumonisins are poorly absorbed at the gastrointestinal level and oral bioavailability generally remains lower than 5% for FB1 (and appears to be even lower for FB2). The absorbed fractions are rapidly distributed and eliminated in the urine. In the study with rats to evaluate the efficacy of the bio-organoclay object of the invention in decreasing the urinary excretion and therefore the bioavailability of FB1, urine samples from the control and treated groups were collected 24, 48 and 72 hours after the administration of 3 mg of FB1 by gastric gavage. Due to the analytical method performance, it was necessary to collect a larger volume of urine sample for toxin detection; for this reason urine samples were collected at 24-hour intervals. Samples collected at 24 hours post-dose showed the highest toxin concentration. At 48 and 72 hours, FB1 concentration decreased by 3- and 5-fold compared to 24-hour samples.

Also for FB1, the use of the rat animal model confirmed the efficacy of the bio-organoclay, object of the invention, in sequestering the toxin *in vivo* and reducing its bioavailability. $FB_1$ urinary excretion values ($AUC_{0 \to 72}$ and $C_{max}$ at 24 hours) in rats treated with 125 mg of bio-organoclay/animal were significantly lower than those measured for animals in the control group (p<0.05). In particular, the bio-organoclay sequestered FB1 in the animals' gastrointestinal tract and reduced its urinary excretion by about 40%. The total excretion of toxin by the animals belonging to the bio-clay treated group was equal to 0.62±0.12% of the administered dose, and this value was significantly lower (p<0.05) than that measured for the animals of the group control (1.18±0.52%).

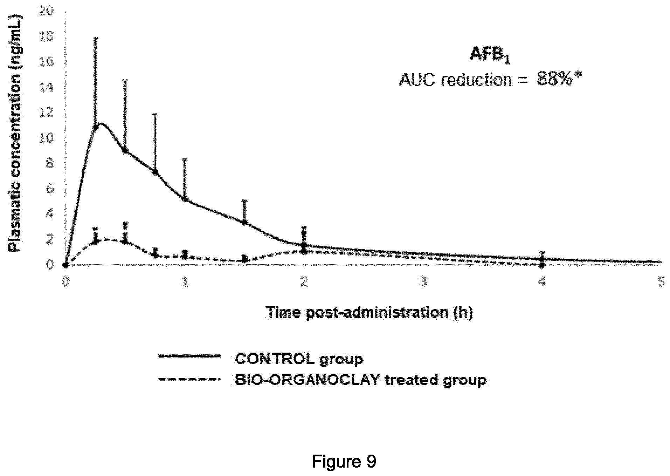

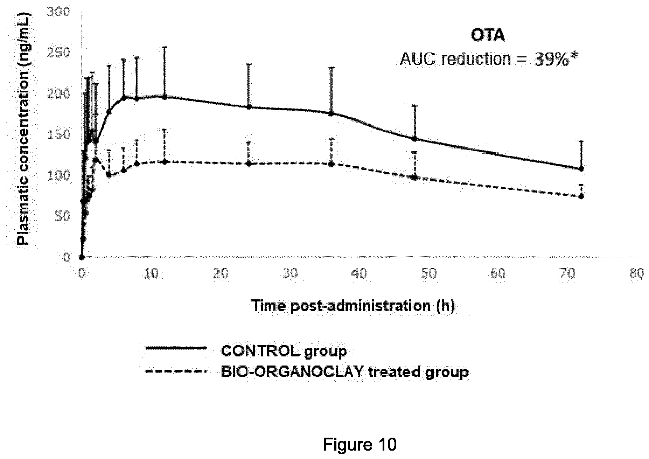

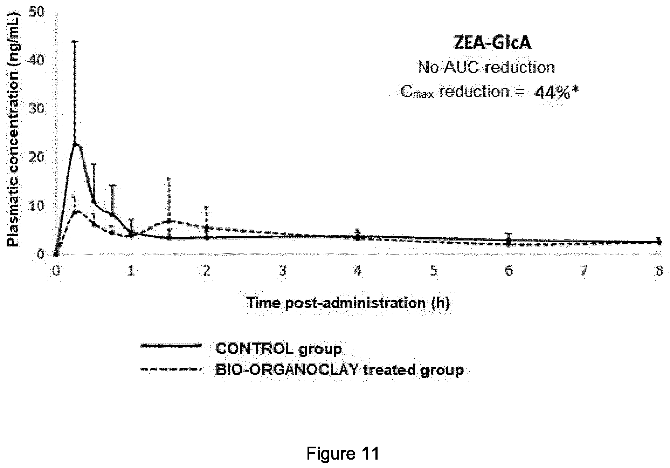

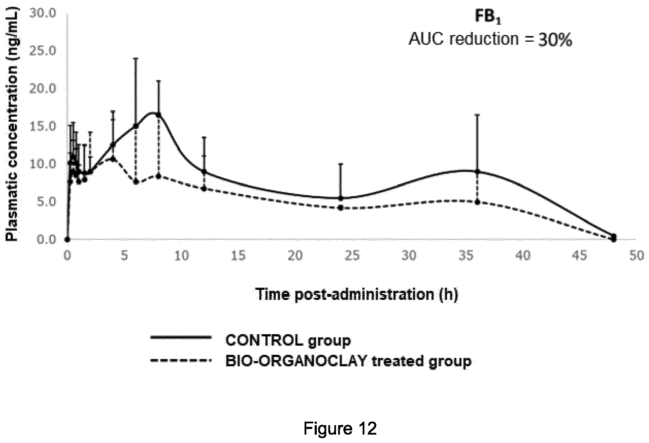

Tables 22 - 25 and Figures 9-12 report the toxicokinetic and plasma concentration results measured in the pig test for animals in the "control" and bio-organoclay "treated" groups.

Table 22. Main toxicokinetic parameters measured for AFB1 after oral administration of the toxin via a single intragastric bolus, containing AFB1 alone (C1 and C2) or in association with the bio-organoclay object of the invention (T1 and T2), to 7 or 8 pigs per subgroup (C1: n=8; T1, C2, T2: n=7). In the C1 and T1 subgroups the feed was suspended from 12 hours before to 4 hours after the administration of the bolus. Values represent the mean +/- SD.

| Toxicokinetic parameter | Feed deprivation | | *Ad libitum* feed | |
|---|---|---|---|---|
| | C1 | T1 | C2 | T2 |
| $AUC_{0 \to \infty}$ (h.ng/mL) | 13.77 ± 7.06 | N.D. | 9.98 ± 5.54 | N.D. |
| $AUC_{0 \to t}$ (h.ng/mL) | 12.60± 7.02* | 1.54 ± 1.27* | 6.80 ± 4.76* | 1.53 ± 1.38* |
| $C_{max}$ (ng/mL) | 11.76 ± 6.93* | 2.01 ± 1.62* | 7.36 ± 5.64 | 3.33 ± 2.22 |
| $T_{max}$ (h) | 0.38 ± 0.13 | 0.67 ± 0.65 | 0.42 ± 0.13 | 1.07 ± 1.36 |
| $T_{1/2\ el}$ (h) | 1.04 ± 0.46 | ND | 0.88 ± 0.44 | ND |
| $k_{ei}$ (1/h) | 0.87 ± 0.64 | ND | 0.93 ± 0.35 | ND |
| Rel. F (%) | 100 | 12.2 | 100 | 22.5 |

*$AUC_{0 \to \infty}$: area under the curve from time zero to infinity; $AUC_{0 \to t}$: area under the curve from time zero to the last sampling point with values above LOQ; $C_{max}$: maximum plasma concentration; $T_{max}$: time to maximum plasma concentration; $T_{1/2}$ el: elimination half-life; $k_{el}$: elimination rate constant; Rel. F: relative oral bioavailability. N.D.: not determined; \* = significant difference between C1 and T1 or C2 and T2.*

The bio-organoclay confirmed its efficacy in sequestering AFB1 also in the gastrointestinal tract of pigs and in reducing its bioavailability. For $AUC_{0 \to t}$, values, in fact, significant differences were found between the C1 and T1 subgroups (p=0.001; two-way ANOVA with post-hoc Tukey HSD test) and between the C2 and T2 subgroups (p=0.01; Kruskal-Wallis), which produced low values for F, related to both treatment groups (12.2% and 22.5% for T1 and T2, respectively). From the data in Table 17, it can be seen that the reduction in $AUC_{0 \to t}$ in both treatment groups is due to a notable reduction in $C_{max}$. A significant difference for $C_{max}$ was found between the C1 and T1 subgroups (p = 0.002; Kruskal-Wallis test), while a trend towards a significant reduction was found between the C2 and T2 subgroups (p = 0.162; two-way ANOVA with post-hoc Tukey HSD test after logarithmic transformation). For all other parameters, for the T1 subgroup compared to the C1 subgroup and for the T2 subgroup compared to the C2 subgroup, no significant differences were found. Furthermore, no significant differences in toxicokinetic parameters were found between the C1 and C2 subgroups, suggesting that the presence of feed has no effect on AFB1 oral absorption in pigs using the oral bolus model.

Table 23. Main toxicokinetic parameters measured for OTA after oral administration of the toxin by a single intragastric bolus, containing OTA alone (C1 and C2) or in association with the bio-organoclay object of the invention (T1 and T2), to 7 or 8 pigs per subgroup (C1: n=8; T1, C2, T2: n=7). In the C1 and T1 subgroups the feed was suspended from 12 hours before to 4 hours after the administration of the bolus. Values represent the mean+/- SD.

| Toxicokinetic parameter | Feed deprivation | | Ad libitum feed | |
|---|---|---|---|---|
| | C1 | T1 | C2 | T2 |
| $AUC_{0 \to 72}$ (h.$\mu$g/mL) | 11.23± 3.17* | 6.86 ± 1.29* | 12.27 ± 4.93* | 9.42 ± 3.01* |
| $C_{max}$ ($\mu$g/mL) | 0.22 ± 0.51* | 0.14 ± 0.04* | 0.24 ± 0.04* | 0.17 ± 0.04* |
| $T_{max}$ (h) | 9.88 ± 7.49 | 19.43± 14.18 | 19.33± 16.72 | 10.93 ± 9.56 |
| Rel. F (%) | 100 | 61.1 | 100 | 76.8 |

$AUC_{0 \to 72}$ area under the curve from time zero to the last sampling point; $C_{max}$: maximum plasma concentration; $T_{max}$: time to maximum plasma concentration; Rel. F: relative oral bioavailability; * = (border-line) significant difference betweenC1 and T1 or C2 and T2

For $AUC_{0 \to 72}$ values, border-line differences were found between the C1 and T1 subgroup (p=0.071; two-way ANOVA with post-hoc Tukey HSD test) and between the C2 and T2 subgroup (p=0.092; two-way ANOVA with post-hoc Tukey HSD test), which produced low values for F and for both treatment groups (61.1% and 76.8% for T1 and T2, respectively). In both treatment groups, the reduction in $AUC_{0 \to 72}$ was determined by a notable and significant reduction in $C_{max}$. For all other parameters, no significant differences were found for the T1 subgroup with respect to the C1 subgroup and for the T2 subgroup with respect to the C2 subgroup. Furthermore, no significant differences in toxicokinetic parameters were found between the C1 and C2 subgroups, suggesting that the presence of feed has no effect on the oral absorption of OTA in pigs, using the oral bolus model.

Table 24. Main toxicokinetic parameters measured for ZEN-GIcA after oral administration of ZEA via a single intragastric bolus, containing ZEA alone (C1 and C2) or in association with the bio-organoclay object of the invention (T1 and T2), to 7 or 8 pigs per subgroup (C1: n=8; T1, C2, T2: n=7). In the C1 and T1 subgroups the feed was suspended from 12 hours before to 4 hours after the administration of the bolus. Values represent the mean +/- SD.

| Toxicokinetic parameter | Feed deprivation | | Ad libitum feed | |
|---|---|---|---|---|
| | C1 | T1 | C2 | T2 |
| $AUC_{0 \to \infty}$(h.ng/mL) | 71.23± 15.45 | 87.96± 39.54 | 76.10± 35.80 | 73.99± 25.48 |
| $AUC_{0 \to t}$(h.ng/mL) | 57.78± 15.76 | 61.16± 15.84 | 56.48± 33.13 | 41.35± 18.87 |
| $C_{max}$(ng/mL) | 22.76± 21.28 | 12.76± 6.73 | 13.34 ± 8.60 | 10.67± 5.63 |
| $T_{max}$(h) | 0.31± 0.18 | 0.71 ± 0.73 | 0.25± 0 | 0.36± 0.20 |
| $T_{1/2\ el}$ (h) | 11.16± 3.02 | 15.94± 11.99 | 12.23± 6.19 | 13.64± 5.66 |
| $k_{el}$ (1/h) | 0.07± 0.02 | 0.08± 0.068 | 0.08± 0.05 | 0.06± 0.03 |
| Rel. F (%) | 100 | 123.5 | 100 | 97.2 |

$AUC_{0 \to \infty}$: area under the curve from time zero to infinity; $AUC_{0 \to t}$ area under the curve from time zero to the last sampling point with values above LOQ; $C_{max}$: maximum plasma concentration; $T_{max}$: time to maximum plasma concentration; $T_{1/2}$ el: elimination half-life; $k_{el}$: elimination rate constant; Rel. F: relative oral bioavailability.

For all parameters, no significant differences were recorded at p<0.05 for the T1 subgroup with respect to the C1 subgroup and for the T2 subgroup with respect to the C2 subgroup. However, lower $C_{max}$ values were recorded for the T1 and T2 subgroups, with respect to the respective controls. No significant differences in toxicokinetic parameters were found between the C1 and C2 subgroups, suggesting that also in this case the presence of feed had no effect on the oral absorption of ZEA.

Table 25. Main toxicokinetic parameters measured for FB1 after oral administration of the toxin via a single intragastric bolus, containing FB1 alone (C1 and C2) or in association with the bio-organoclay object of the invention (T1 and T2), to 7 or 8 pigs per subgroup (C1: n=8; T1, C2, T2: n=7). In the C1 and T1 subgroups the feed was suspended from 12 hours before to 4 hours after the administration of the bolus. Values represent the mean +/- SD.

| Toxicokinetic parameter | Feed deprivation | | Ad libitum feed | |
|---|---|---|---|---|
| | C1 | T1 | C2 | T2 |
| $AUC_{0\rightarrow t}$(h.$\mu$g/mL) | 0.22 ± 0.18 | 0.16 ± 0.12 | 0.14 ± 0.05 | 0.18 ± 0.14 |
| $C_{max}$(ng/mL) | 18.78 ± 6.48 | 12.71 ± 6.63 | 19.90± 13.85 | 17.69 ± 8.45 |
| $T_{max}$(h) | 5.75 ± 2.85 | 3.00 ± 2.60 | 2.75 ± 3.01 | 3.07 ± 2.99 |
| Rel. F (%) | 100 | 69.8 | 100 | 125.1 |
| $AUC_{0\rightarrow t}$: area under the curve from time zero to the last sampling point; $C_{max}$: maximum plasma concentration; $T_{max}$: time to maximum plasma concentration; Rel. F: relative oral bioavailability. | | | | |

For all parameters, no significant differences were found with values of p<0.05, for the T1 subgroup with respect to the C1 subgroup and for the T2 subgroup with respect to the C2 subgroup. However, a decreasing trend was observed for the AUC values for the T1 group with respect to the C1 group, by about 30%, indicative of a lower bioavailability of the toxin in the animals of the treated group. Finally, no significant differences in toxicokinetic parameters were found between the C1 and C2 subgroups, suggesting that the presence of feed had no effect on FB1oral absorption in pigs.

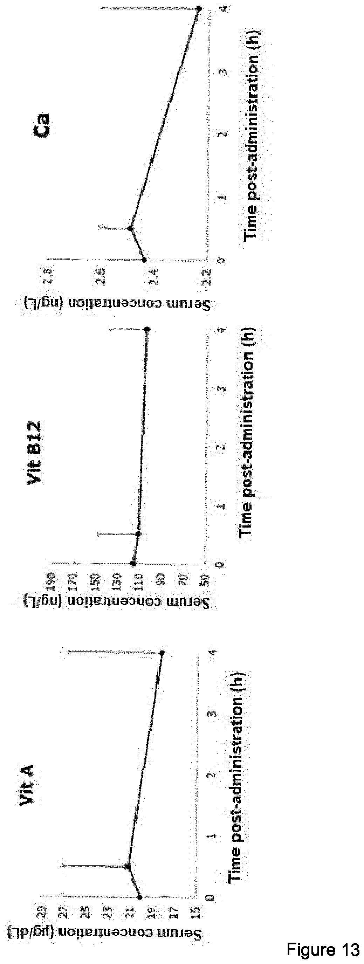

Finally, as regards the effect of the bio-organoclay on the bioavailability of micronutrients, as seen in Figure 13, no significant differences were found with values of p<0.05 for the serum concentrations of Vit A, Vit B12 and Ca at time 0 (before the administration of the mycotoxins and bio-organoclay) with respect to the serum concentrations of Vit A, Vit B12 and Ca at time 0.5 h and at time 4 hours after administration.

In conclusion, with regards to the micronutrients and the safety of use of the bio-organoclay object of the invention, it can be stated that the bio-organoclay did not significantly alter the serum concentrations of Vit A, Vit B12 and Ca after the administration of a single dose of 0.25 g of additive/kg of body weight.

**Claims**

1.   A process for the preparation of an organophilic smectite comprising the following steps:

   a) supplying a sodium smectite of sedimentary geological origin;
   b) treating said smectite with $H_2SO_4$ in an amount in the range from 10% to 30% by weight with respect to the smectite dry weight, at a temperature in the range from 20°C to 50°C, to obtain an activated smectite;
   c) treating said activated smectite with at least one modifying agent consisting of a quaternary ammonium salt of formula [$(CH_3)_3N^+(CH_2)_2OH$] $X^-$, where $X^-$ is a monovalent anion,
   where said modifying agent is at a concentration in the range from 30 to 130 meq/100g to obtain a functionalized smectite; and
   d) extruding the functionalized smectite to reach an extrudate temperature equal to or higher than 45°C, and drying it at a temperature in the range from 30°C to 80°C.

2.   The process according to claim 1, wherein the smectite of step a) is in a crude form and contains an amount of montmorillonite equal to or higher than 70% and moisture at 20%.

3.   The process according to claim 1 or claim 2, wherein $H_2SO_4$ is in an amount of about 15% by weight with respect to the smectite dry weight.

4.   The process according to any one of claims 1 to 3, wherein in step c) the at least one modifying agent is a quaternary ammonium salt of formula [$(CH_3)_3N^+(CH_2)_2OH$] X-, where X- is selected from the group consisting of chloride, hydroxide anion, bitartrate and dihydrogen citrate, preferably chloride.

5.   The process according to claim 4, wherein in step c) the modifying agent is choline chloride at a concentration in the range from 65 to 75 meq/100g.

6. The process according to any one of claims 1 to 5, wherein the drying step following the extrusion is carried out in an oven at a temperature of about 65°C.

7. An organophilic smectite obtainable from the process according to any one of claims 1 to 6, wherein said organophilic smectite has an acidic character and comprises at least one organic modifier consisting of choline in the mineral interlayer.

8. Use of the organophilic smectite of claim 7 for removing mycotoxins by adsorption.

9. The use according to claim 8, wherein said mycotoxins are selected from the group consisting of aflatoxin B1 (AFB1), zearalenone (ZEA), ochratoxin A (OTA), and fumonisin B1 (FB1).

10. The use according to claim 9, wherein the organophilic smectite is capable of simultaneously sequestering, *in vitro,* from 10% to 100% of AFB1, FB1, OTA and ZEA over a broad range of pH values (3-9), at a concentration of 1 ppm for each toxin and at dosages of smectite equal to or higher than 0.1% w/v.

11. The use according to claim 9, wherein the organophilic smectite is capable of simultaneously sequestering AFB1, ZEA, OTA and FB1, with maximum adsorption capacity values (expressed as $\mu$g of adsorbed toxin per mg of smectite) between 30-150 $\mu$g/mg for AFB1, 20-150 $\mu$g/mg for FB1, 20-60 $\mu$g/mg for ZEA and 4-20 $\mu$g/mg for OTA.

12. A food product or a supplement comprising the smectite of claim 7 as an additive.

13. The food product according to claim 12, wherein said food product is an animal feed.

14. The food product according to claim 12 or 13, wherein the smectite is present in the product as an additive at dosages equal to or higher than 0.25% w/v.

15. A food product or a supplement comprising the smectite of claim 7 for use in the treatment or prevention of myco-toxicosis.

Figure 1

Figure 2

Figure 3

Figura 4

38

Figure 5

Figure 6

Figure 7

Figure 8

Figure 9

Figure 10

Figure 11

Figure 12

Figure 13

Europäisches
Patentamt

European
Patent Office

Office européen
des brevets

# EUROPEAN SEARCH REPORT

Application Number

EP 24 15 1099

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 0 133 071 A2 (CECA SA [FR]) 13 February 1985 (1985-02-13) * the whole document * ----- | 1-15 | INV. C09C1/42 A61K33/06 A61K33/12 |
| A | US 2021/137971 A1 (PHILLIPS TIMOTHY D [US] ET AL) 13 May 2021 (2021-05-13) * paragraphs [0072] – [0110], [0116] * ----- | 1-15 | |

TECHNICAL FIELDS
SEARCHED       (IPC)

C09C
A61K

The present search report has been drawn up for all claims

| Place of search | Date of completion of the search | Examiner |
|---|---|---|
| The Hague | 12 April 2024 | Siebel, Eric |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
...........................................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 24 15 1099

This annex lists the patent family members relating to the patent documents cited in the above-mentioned European search report.
The members are as contained in the European Patent Office EDP file on
The European Patent Office is in no way liable for these particulars which are merely given for the purpose of information.

12-04-2024

| Patent document cited in search report | | Publication date | Patent family member(s) | | Publication date |
|---|---|---|---|---|---|
| EP 0133071 | A2 | 13-02-1985 | EP | 0133071 A2 | 13-02-1985 |
| | | | FR | 2547826 A1 | 28-12-1984 |
| US 2021137971 | A1 | 13-05-2021 | AU | 2019326455 A1 | 04-03-2021 |
| | | | CA | 3110252 A1 | 27-02-2020 |
| | | | EP | 3840590 A1 | 30-06-2021 |
| | | | JP | 7384455 B2 | 21-11-2023 |
| | | | JP | 2021534971 A | 16-12-2021 |
| | | | JP | 2024016144 A | 06-02-2024 |
| | | | KR | 20230026927 A | 27-02-2023 |
| | | | US | 2021137971 A1 | 13-05-2021 |
| | | | WO | 2020041379 A1 | 27-02-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20210137971 A1, Phillips

**Non-patent literature cited in the description**

- **JIAQI MAO 1,† ; YING ZHOU 2,† ; GUANGLIE LV 1 ; RENXIAN ZHOU 1.** Simultaneous Detoxification of Aflatoxin B1, Zearalenone and Deoxynivalenol by Modified Montmorillonites. *Molecules,* 2022, vol. 27 (1), 315, https://doi.org/10.3390/molecules27010315

- **WILLIAM F. JAYNES ; RICHARD E. ZARTMAN.** Aflatoxin Toxicity Reduction in Feed by Enhanced Binding to Surface-Modified Clay Additives. *Toxins (Basel).,* June 2011, vol. 3 (6), 551-565